Europäisches Patentamt
European Patent Office
Office européen des brevets

(11) Publication number: 0 550 775 A1

# EUROPEAN PATENT APPLICATION

(21) Application number: 92100119.4

(22) Date of filing: 07.01.92

(51) Int. Cl.5: C07D 501/46, C07D 501/42, A61K 31/545, C07D 501/44

(43) Date of publication of application: 14.07.93 Bulletin 93/28

(84) Designated Contracting States: PT

(71) Applicant: F. HOFFMANN-LA ROCHE AG
Postfach 3255
CH-4002 Basel(CH)

(72) Inventor: Albrecht, Harry Allen
139 Waughaw Road
Towaco, N. J. 07082(US)
Inventor: Keith, Dennis Dalton
8 Mendl Terrace
Montclair, N.J. 07042(US)
Inventor: Wei, Chung-Chen
244 Malapardis Road
Cedar Knolls, N.J. 07927(US)

(74) Representative: Martin, Björn et al
Grenzacherstrasse 124 Postfach 3255
CH-4002 Basel (CH)

(54) **Cephalosporin derivatives.**

(57) Cephalosporin derivatives of the general formula

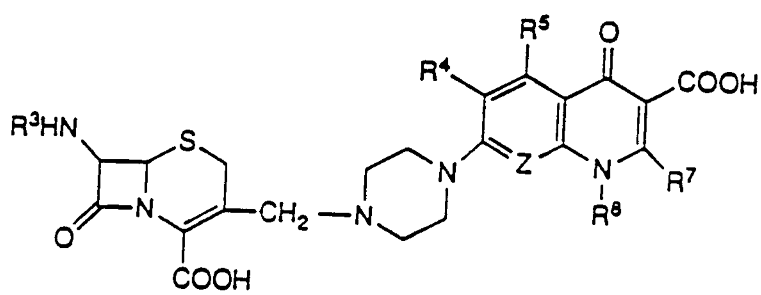

I

in which $R^3$ is a group of the formula

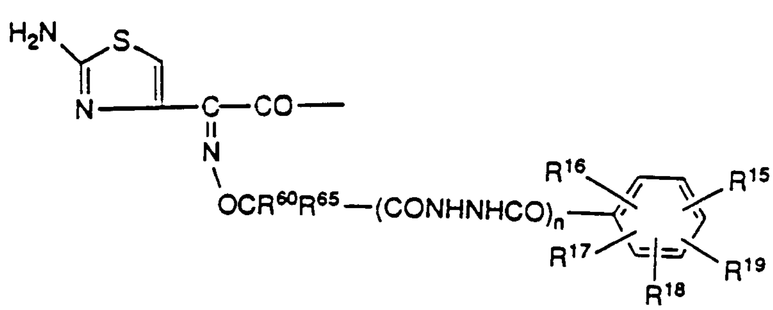

(a)

$R^{60}$ and $R^{65}$ are hydrogen or lower alkyl, $R^{15}$-$R^{19}$ are each independently hydrogen, halogen or hydroxy, n is zero or 1, $R^4$ and $R^5$ are each hydrogen, halogen, lower alkyl, lower alkoxy or amino, $R^7$ is hydrogen or lower

EP 0 550 775 A1

alkyl and $R^8$ is lower alkyl, halo- lower alkyl, $C_3$-$C_7$ cycloalkyl or mono-, di- or trihalophenyl, and Z is N or C-$R^9$, where $R^9$ is hydrogen, halogen, lower alkyl or lower alkoxy; and, when $R^4$ is fluoro, $R^5$ and $R^7$ are hydrogen. $R^8$ is cyclopropyl and Z is C-H, $R^3$ can also be one of the groups

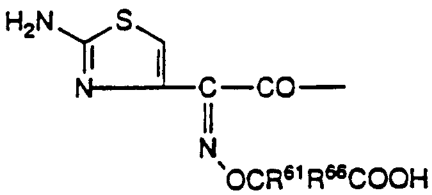

(b)

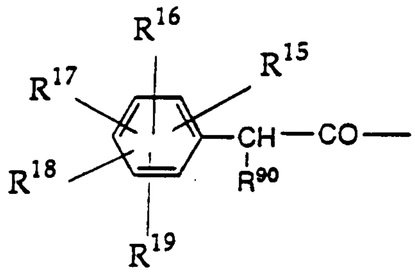

(c)

in which $R^{61}$ and $R^{66}$ are hydrogen or methyl, $R^{15}$-$R^{19}$ are as above and $R^{90}$ is hydrogen, hydroxy, carboxy, amino or [[4-(lower alkyl)-2,3-dioxo-1-piperazinyl]carbonyl]amino;
pharmaceutically acceptable acid or base addition salts thereof, hydrates of compounds of formula I and hydrates of such salts.

The products have antibacterial activity.

This invention relates to cephalosporin derivatives of the general formula

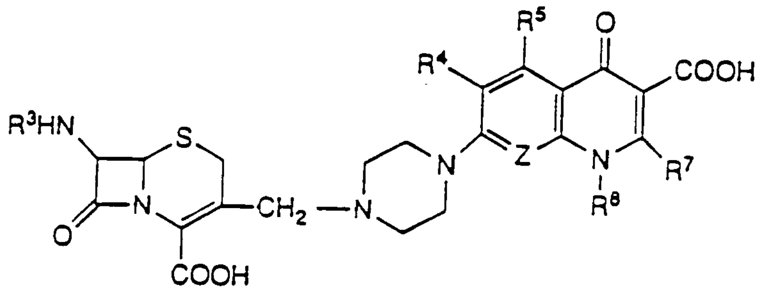

I

in which $R^3$ is a group of the formula

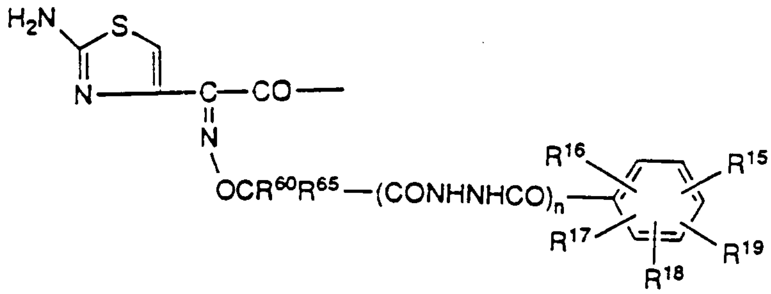

(a)

$R^{60}$ and $R^{65}$ are hydrogen or lower alkyl, $R^{15}$-$R^{19}$ are each independently hydrogen, halogen or hydroxy, n is zero or 1, $R^4$ and $R^5$ are each hydrogen, halogen, lower alkyl, lower alkoxy or amino, $R^7$ is hydrogen or lower alkyl and $R^8$ is lower alkyl, halo- lower alkyl, $C_3$-$C_7$ cycloalkyl or mono-, di- or trihalophenyl, and Z is N or C-$R^9$, where $R^9$ is hydrogen, halogen, lower alkyl or lower alkoxy; and, when $R^4$ is fluoro, $R^5$ and $R^7$ are hydrogen, $R^8$ is cyclopropyl and Z is C-H, $R^3$ can also be one of the groups

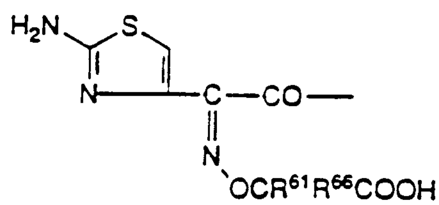

(b)

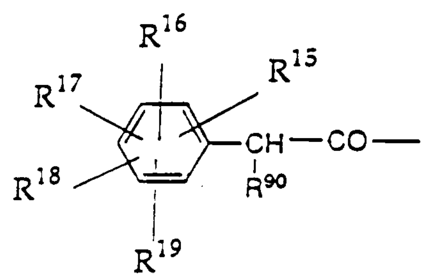

(c)

in which $R^{61}$ and $R^{66}$ are hydrogen or methyl, $R^{15}$-$R^{19}$ are as above and $R^{90}$ is hydrogen, hydroxy, carboxy, amino or [[4-(lower alkyl)-2,3-dioxo-1-piperazinyl]carbonyl]amino;
pharmaceutically acceptable acid or base addition salts thereof, hydrates of compounds of formula I and hydrates of such salts.

The compounds of the present invention can be used for the treatment and prevention of bacterial infections in mammals, both humans and non-humans. Examples of bacterial infections for which the compounds of the present invention are useful in treating include urogenital infections and respiratory infections. The compounds of the present invention exhibit antibacterial activity against a broad range of both gram-negative and gram-positive bacteria.

As used herein, "alkyl" refers to both straight and branched chain saturated hydrocarbon groups having 1 to 8 carbon atoms. "Lower alkyl" refers to those alkyl groups having 1 to 4 carbon atoms. Examples of lower alkyl groups and alkyl groups include methyl, ethyl, n-propyl, isopropyl, t-butyl, n-hexyl, and the like.

As used herein, "alkoxy" refers to a straight or branched chain hydrocarbonoxy group wherein the alkyl portion thereof is alkyl as defined hereinabove. "Lower alkoxy" refers to straight or branched chain hydrocarbonoxy group wherein the lower alkyl portion thereof is lower alkyl as defined above. Examples of lower alkoxy groups and alkoxy groups include methoxy, ethoxy, propoxy, pentoxy, and the like.

As used herein, "cycloalkyl" refers to cyclic hydrocarbons having 3 to 7 carbon atoms, for example, cyclopropyl and cyclohexyl. The carbon atoms that form the cycloalkyl ring can be unsubstituted or substituted.

As used herein, "halo" or "halogen" refers to bromo or bromine; chloro or chlorine; fluoro or fluorine; or iodo or iodine, respectively.

Especially preferred compounds of formula I and their salts and hydrates are those, wherein Z is C-$R^9$ where $R^9$ is hydrogen or halogen. Particularly preferred are those wherein $R^4$ is fluoro, $R^5$ and $R^7$ are hydrogen, $R^8$ is cyclopropyl and Z is C-H, i.e. where the quinolone moiety has the formula

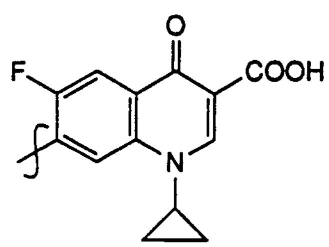

Preferred groups $R^3$ are of the formulas

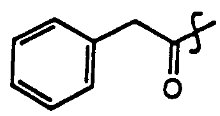

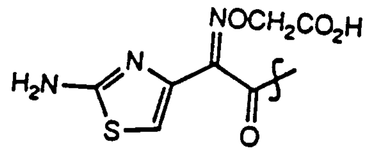

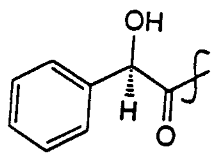

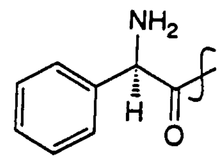

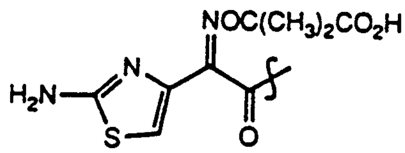

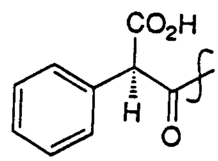

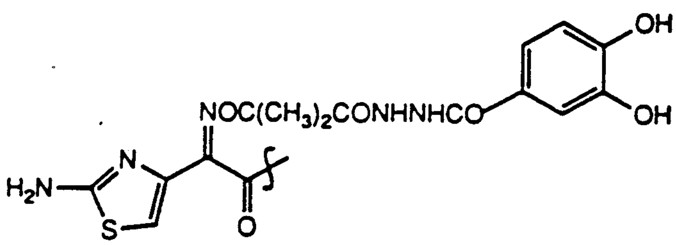

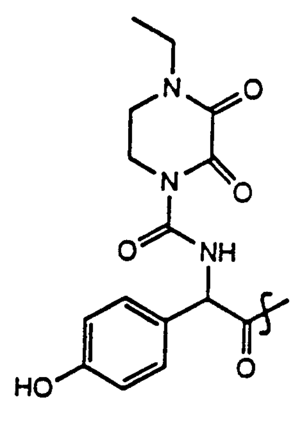

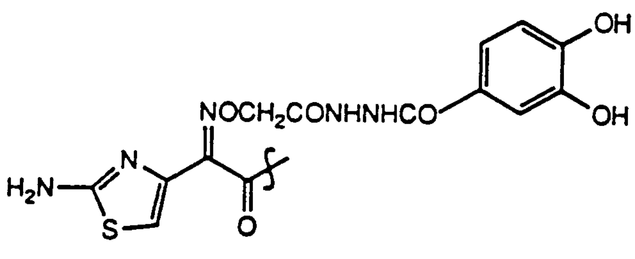

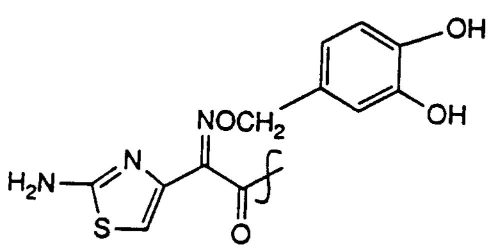

Examples of pharmaceutically acceptable base addition salts of the compounds of the invention include salts with cations derived from metals, quaternary ammonium salts derived from organic bases and amino acid salts. Examples of preferred metal cations are those derived from the alkali metals, for example, lithium, sodium and potassium, and from the alkaline earth metals, for example, calcium and magnesium, although cationic forms of other metals, such as iron, aluminium and zinc, are within the scope of this invention. Examples of cations derived from quaternary ammonium salts derived from organic bases include

tetramethylammonium, tetraethylammonium, benzyltrimethylammonium, phenyltriethylammonium and the like. Other base addition salts are the salts with organic bases such as salts with amines (for example, salts with N-ethyl-piperidine, procaine, dibenzylamine, N,N-dibenzylethylenediamine, alkylamines or dialkylamines) as well as salts with amino acids such as, for example, salts with arginine or lysine.

The compounds of the invention, when they contain a basic functional group such as an amine, also form addition salts with organic or inorganic acids. Examples of such salts are hydrohalides (for example, hydrochlorides, hydrobromides and hydroiodides) as well as other mineral acid salts such as sulfates, nitrates, phosphates and the like, alkylsulfonates and monoarylsulfonates such as ethanesulfonates, toluenesulfonates, benzenesulfonates and the like and also other organic acid salts such as acetates, trifluoroacetates, tartrates, maleates, citrates, benzoates, salicylates, ascorbates and the like.

The compounds of the invention as well as their salts and readily hydrolyzable esters can be hydrated. The hydration can be effected in the course of the manufacturing process or can occur gradually as a result of hygroscopic properties of an initially anhydrous product.

Specifically preferred compounds of the present invention include:

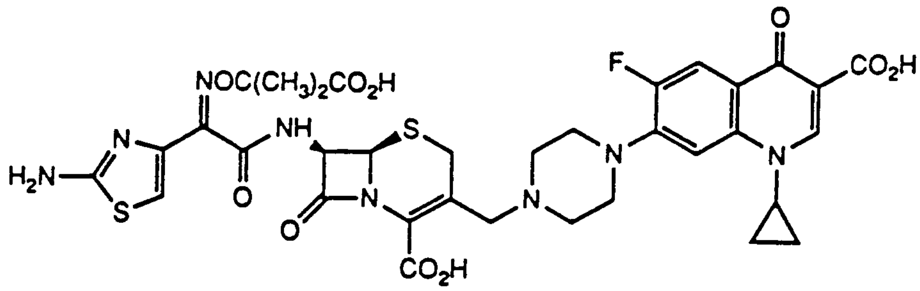

A

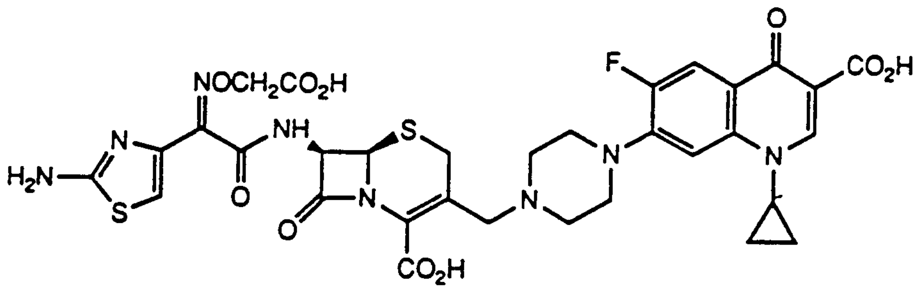

B

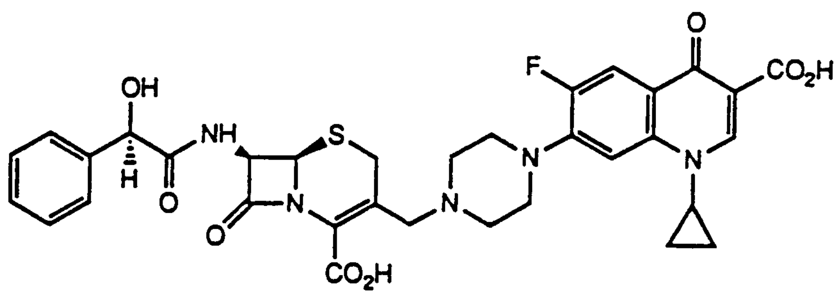

C

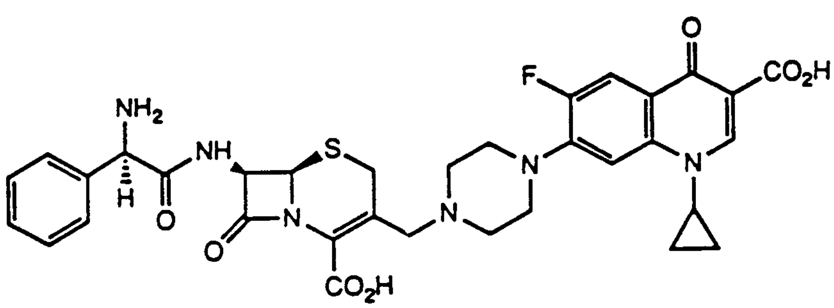

D

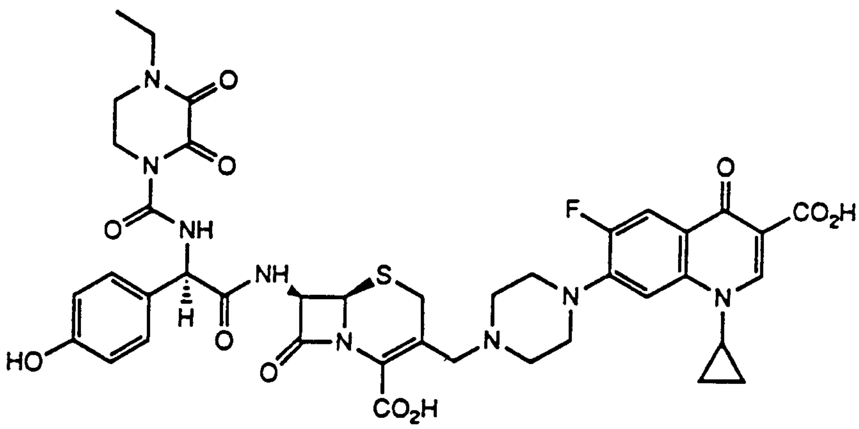

E

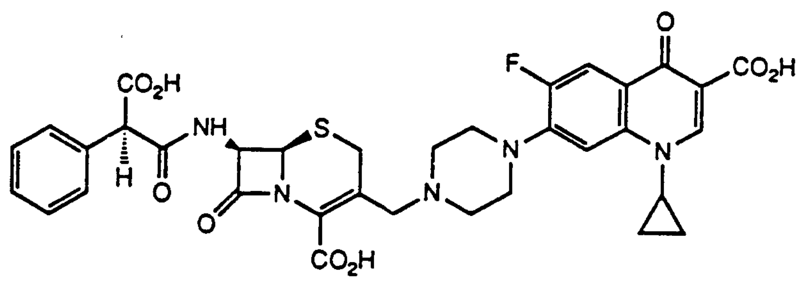

F

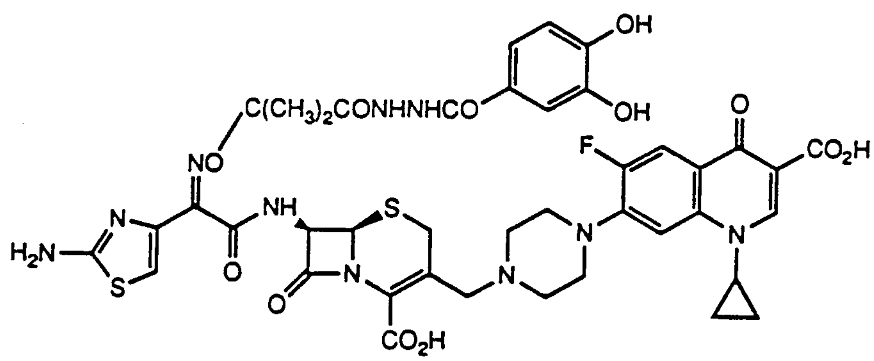

G

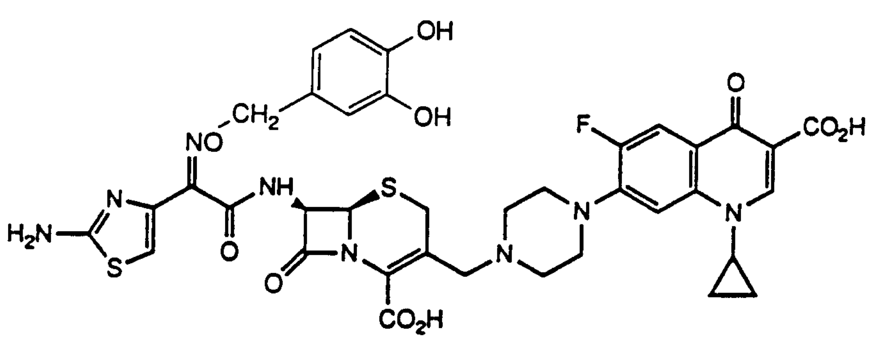

H

and pharmaceutically acceptable acid or base addition salts thereof.

In vitro activity of the compounds of the present invention was measured by the Minimum Inhibitory Concentration (MIC) in micrograms/ml utilizing the Agar Dilution Method, which is well known in the art, against a variety of gram-negative and gram-positive organisms. The data are set forth in the table below.

Compounds of the present invention tested:

Compound A:

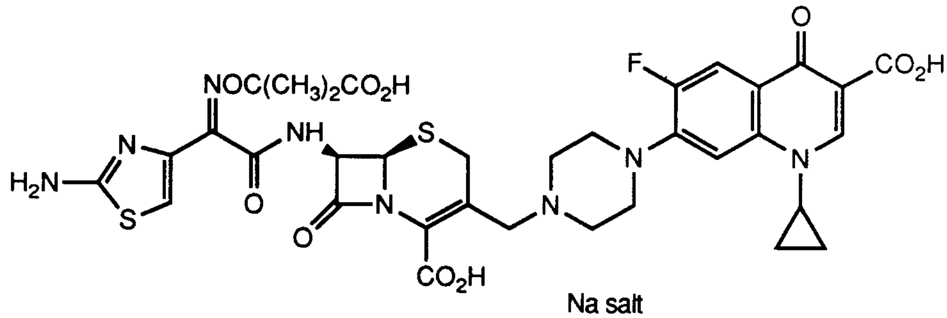

Na salt

[6R-[6α,7β(Z)]]-7-[[(2-Amino-4-thiazolyl)[(1-carboxyl-1-methylethoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]-oct-2-ene-2-carboxylic acid sodium salt.

Compound B:

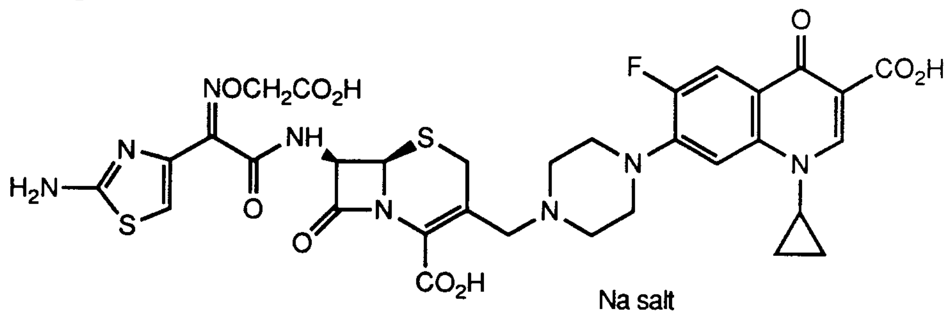

Na salt

[6R-[6α,7β(Z)]]-7-[[(2-Amino-4-thiazolyl)[(carboxymethoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]-oct-2-ene-2-carboxylic acid sodium salt.

Compound C:

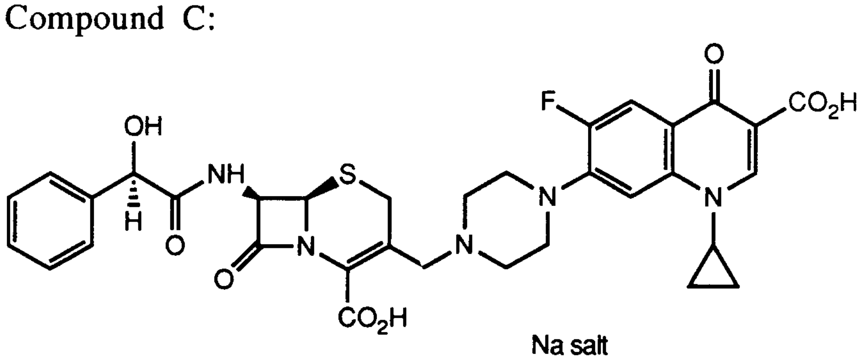

Na salt

[6R-[6α,7β(R)]]-3-[[4-(3-Carboxy-1-cyclopropyl-6-fluoro-1,4-dihydro-4-oxo-7-quinolinyl)-1-piperazinyl]methyl]-7-[(hydroxyphenylacetyl)amino]-8-oxo-5-thia-1-azabicyclo[4.2.0]oct-2-ene-2-carboxylic acid sodium salt.

Compound D:

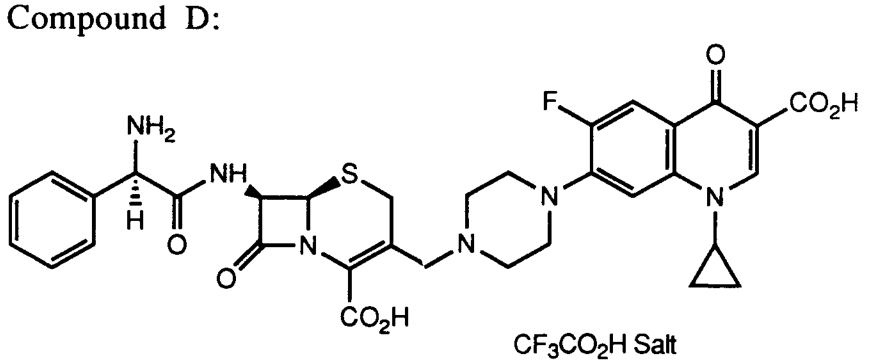

$CF_3CO_2H$ Salt

[6R-[6α,7β(R)]]-7-[(Aminophenylacetyl)amino]-3-[[4-(3-carboxyl-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid trifluoroacetic acid salt.

Compound E:

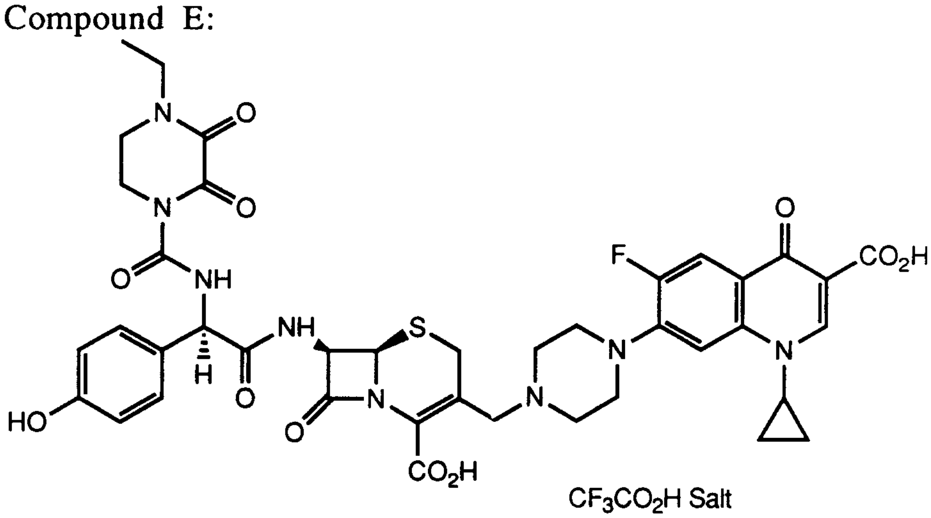

$CF_3CO_2H$ Salt

[6R-[6a,7β(R)]]-3-[[4-(3-Carboxy-1-cyclopropyl-6-fluoro-1,4-dihydro-4-oxo-7-quinolinyl)-1-piperazinyl]methyl]-7-[[[[(4-ethyl-2,3-dioxo-1-piperazinyl)carbonyl]amino](4-hydroxyphenyl)acetyl]amino]-8-oxo-5-thia-1-azabicyclo[4.2.0]oct-2-ene-2-carboxylic acid trifluoroacetic acid salt.

Compound F:

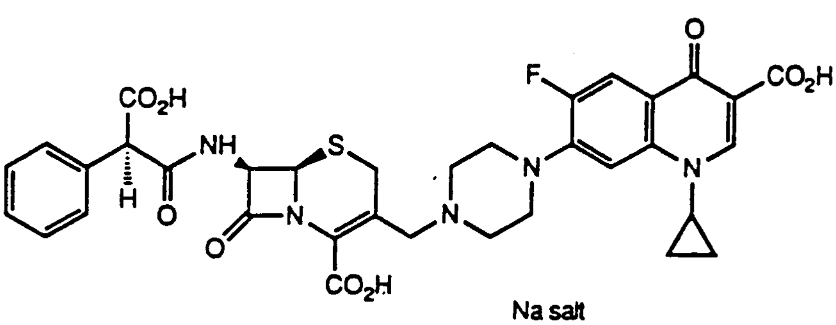

Na salt

(6R-trans)-3-[[4-(3-Carboxy-1-cyclopropyl-6-fluoro-1,4-dihydro-4-oxo-7-quinolinyl)-1-piperazinyl]methyl]-7-[-(carboxyphenylacetyl)amino]-8-oxo-5-thia-1-azabicyclo[4.2.0]oct-2-ene-2-carboxylic acid sodium salt.

Compound G:

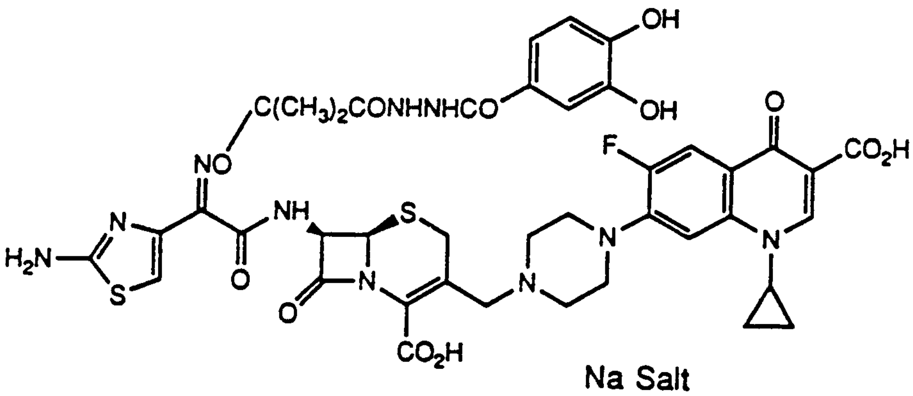

Na Salt

[6R-[6α,7β(Z)]]-7-[[[[(2-Amino-4-thiazolyl)[1-[(3,4-dihydroxybenzoyl)hydrazino]carbonyl]-1-methylethoxy]-imino]acetyl]amino]-3-[[4-(3-carboxy-1-cyclopropyl-6-fluoro-1,4-dihydro-4-oxo-7-quinolinyl)-1-piperazinyl]-methyl]-8-oxo-5-thia-1-azabicyclo[4.2.0]oct-2-ene-2-carboxylic acid sodium salt.

IN VITRO MIC (mg/ml) AGAR DILUTION METHOD

| CULTURE | COMPOUNDS A | B | C | D | E | F | G |
|---|---|---|---|---|---|---|---|
| E. coli 257 | 0.25 | 0.25 | 0.125 | 0.0313 | ≤0.0157 | 0.25 | 0.125 |
| E. coli ATCC 25922 | 0.25 | 0.5 | 0.25 | 0.0313 | ≤0.0157 | 0.25 | 0.125 |
| E. coli TEM-1 | 0.5 | 0.5 | 0.125 | 0.0313 | 0.0313 | 0.25 | 0.0625 |
| Cit. freundi BS-16 | 0.125 | 1 | 0.125 | 0.0625 | 0.0625 | 0.0625 | 0.25 |
| K. pneumoniae A | 0.5 | 1 | 0.5 | 0.0313 | 0.5 | 1 | 0.5 |
| Enter. cloacae 5699 | 1 | 1 | 0.25 | 0.125 | 0.125 | 0.5 | 0.25 |
| Enter. cloacae P99 | 0.5 | 0.25 | 0.0313 | 0.0313 | ≤0.0157 | 0.0625 | 0.25 |
| Ser. marcescens SM | 1 | 1 | 0.25 | 0.125 | 0.25 | 1 | 0.5 |
| Ser. marcescens 1071 | 1 | 2 | 0.25 | 0.125 | 0.25 | 0.5 | 0.5 |
| Prot. vulgaris ATCC 6380 | 0.5 | 0.25 | 0.25 | 0.0625 | 0.125 | 0.5 | 0.125 |
| Prot. vulgaris 1028 BC | 1 | 1 | 0.0625 | 0.0625 | 0.0625 | 0.25 | 0.25 |
| Prot. mirabilis 90 | 2 | 2 | 1 | 0.25 | 1 | 1 | 0.5 |
| Ps. aeruginosa ATCC 27853 | 8 | 16 | 8 | 1 | 8 | 8 | 0.5 |
| Ps. aeruginosa 5712 | 16 | 32 | 16 | 4 | 32 | 16 | 0.25 |
| Ps. aeruginosa 8780 | 8 | 8 | 2 | 1 | 8 | 8 | 0.25 |
| Ps. aeruginosa 765 | 8 | 8 | 2 | 2 | 4 | 8 | 0.5 |
| Ps. aeruginosa 18SH | 2 | 4 | 2 | 1 | 2 | 4 | 0.5 |
| Staph. aureus Smith | 8 | 8 | 0.5 | 0.5 | 2 | 2 | 2 |
| Staph. aureus ATCC 29213 | 8 | 8 | 1 | 1 | 2 | 4 | 4 |
| Staph. aureus 1059B | 4 | 2 | 1 | 1 | 2 | 1 | 4 |
| Staph. aureus 67 | 16 | 32 | 8 | 2 | 16 | 8 | 8 |
| Staph. aureus 753 | 8 | 8 | 1 | 2 | 2 | 2 | 4 |
| Str. pneumoniae 6301 | 0.25 | 0.25 | 0.0625 | 2 | 0.125 | 0.5 | 0.125 |
| Str. pyogenes 4 | 0.25 | 0.125 | 0.0313 | 1 | 0.125 | 0.5 | 0.25 |
| Str. faecalis ATCC 29212 | 8 | 16 | 16 | 4 | 16 | 32 | 4 |

Chemical stability (half-life) in phosphate buffer (pH 7.40; 37°C) of some of the compounds of the present invention was determined using high performance liquid chromatography (HPLC). A Hamilton PRP-1 (250 mm x 4.1 mm) column was used with UV detection at 280 nm. The mobile phase used consisted of a 0.01 M solution of tetradecyltrimethylammonium bromide in a mixture of 70% 0.072M phosphate buffer (pH 8.2) and 30% acetonitrile. In some experiments, minor adjustments to the pH and in the acetonitrile concentration were made to improve resolutions. To determine stability the decrease in the integration of the product peak was monitored at least until it reached 50% of the original value; that is, through one half-life. Semi-logarithmic plots of product peak integrations versus time were essentially linear.

| Chemical Stability (Half-life; phosphate buffer (pH7.40; 37 ° C) of Selected Compounds | |
|---|---|
| Compound | Half life, days |
| A | 12.5 |
| C | 5.5 |
| E | 2.5 |
| F | 9.5 |

For treating or preventing bacterial infections in mammals, both humans and non-humans, a compound of the present invention can be administered to the mammal in need of such treatment or prevention of such infections an amount of the compound from about 5 mg/kg/day to about 500 mg/kg/day, preferably from about 10 mg/kg/day to about 100 mg/kg/day, and most preferably from about 10 mg/kg/day to about 55 mg/kg/day.

Modes of administration which are well known in the art and which are useful in administering penicillins and cephalosporins to the site of the infection are also contemplated as being useful in administering the compounds of the present invention to mammalian hosts, both humans and non-humans. Such modes of administration include intravenous, intramuscular, and enterally, for example, as a suppository. Appropriate therapeutically inert carriers useful for the different modes of administration can also be included, as well as other pharmaceutical adjuvants which are well known in the art.

Suitable carrier materials for intramuscular or intravenous injection solution unit dosage forms are, for example, water, alcohols, polyols, glycerol and vegetable oils. Suitable carrier materials for suppositories are, for example, natural or hardened oils, waxes, fats and semi-liquid or liquid polyols.

The usual stabilizing, preserving, wetting, emulsifying agents, consistency-improving agents, salts for varying the osmotic pressure, buffer substances, solubilizers, and antioxidants also come into consideration as pharmaceutical adjuvants.

The compounds of formula I, their corresponding pharmaceutically acceptable acid or base addition salts, hydrates of compounds of formula I and hydrates of said salts can be manufactured in accordance with the invention by a process which comprises

(a) reacting a compound of the general formula

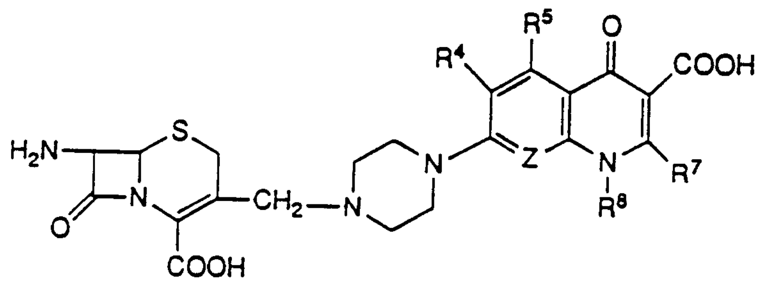

II

wherein $R^4$, $R^5$, $R^7$, $R^8$ and Z are as above,

or a salt thereof with a carboxylic acid of the general formula $R^3OH$ or a reactive derivative thereof, or

(b) converting a compound corresponding to formula I, in which at least one of carboxy, amino and hydroxy groups is protected, to a compound of formula I, or

(c) for the manufacture of a compound of formula I, in which $R^3$ is group (a), reacting a compound of the general formula

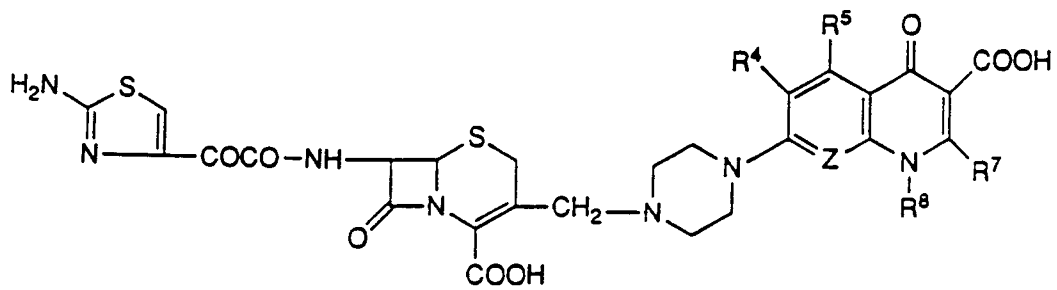

III

wherein $R^4$, $R^5$, $R^7$, $R^8$ and Z are as above,
or a salt thereof with a compound of the general formula

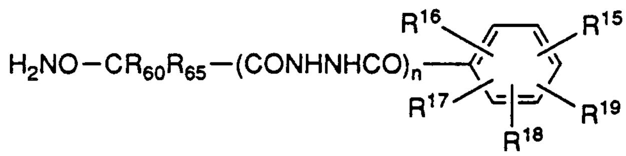

IV

wherein $R^{15}$-$R^{19}$, $R^{60}$, $R^{65}$ and n are as above,
or with a salt thereof, or

(d) for the manufacture of pharmaceutically acceptable acid or base addition salts or hydrates of compounds of formula I or hydrates of such salts converting a compound of formula I into such salt or hydrate or into a hydrate of such salt.

The reaction of compounds II with the acids $R^3OH$ or their reactive derivatives according to embodiment (a) can be carried out in a manner known per se. The carboxy group in compounds II can be protected; for example, by esterification to form a readily cleavable ester such as a silyl ester (e.g. the trimethylsilyl ester). The carboxy group can also be protected by the protecting ester groups described below for embodiment (b). Furthermore, the carboxy group can be protected by salt formation with an inorganic or tertiary organic base such a triethylamine. Amino groups present in groups $R^3$ can be protected. Possible protecting groups are, for example, protecting groups which are cleavable by acid hydrolysis (e.g. the tert.butoxycarbonyl or trityl groups) or by basic hydrolysis (e.g. the trifluoroacetyl group). Other protecting groups are the chloroacetyl, bromoacetyl and iodoacetyl groups, especially the chloroacetyl group. These last-mentioned protecting groups can be cleaved off by treatment with thiourea. The 7-amino group in compounds II can be protected, for example, by a silyl protecting groups such as the trimethylsilyl group.

Examples of reactive functional derivatives of acids of formula $R^3OH$ are halides (i.e. chlorides, bromides and fluorides), azides, anhydrides, especially mixed anhydrides with strong acids, reactive esters (e.g. N-hydroxysuccinimide esters) and amides (e.g. imidazolides).

In reacting a 7-amino compound of formula II with an acid of formula $R^3OH$ or a reactive functional derivative thereof, for example, a free acid of formula $R^3OH$ can be reacted with an aforementioned ester of a compound of formula II in the presence of a carbodiimide such as dicyclohexylcarbodiimide in an inert solvent such as ethyl acetate, acetonitrile, dioxan, chloroform, methylene chloride, benzene or dimethylformamide, and subsequently the ester group can be cleaved off. Oxazolium salts (e.g. N-ethyl-5-phenylisoxazolium-3'-sulphonate) can be used in place of carbodiimides in the foregoing reaction.

According to another embodiment, a salt of an acid of formula II (e.g. a trialkylammonium salt such as the triethylammonium salt) is reacted with a reactive functional derivative of an acid of formula $R^3OH$ in one of the aforementioned inert solvents.

According to a further enbodiment, an acid halide, preferably the chloride, of an acid of formula $R^3OH$ is reacted with an amine of formula II. The reaction is preferably carried out in the presence of an acid-binding agent, for example in the presence of aqueous alkali, preferably sodium hydroxide, or in the presence of an alkali metal carbonate such as potassium carbonate or in the presence of a lower alkylamine such as triethylamine. As the solvent there is preferably used water, optionally in admixture with an inert organic solvent such as tetrahydrofuran or dioxan. The reaction can also be carried out in an aprotic organic solvent such as dimethylformamide, dimethyl sulphoxide or hexamethylphosphoric acid triamide. When a silylated compound of formula II is used, the reaction is carried out in an anhydrous medium.

Advantageous alternatives for acylation involves the use of a 2-benzothiazolyl thioester or a 1-hydroxybenzotriazole ester of the acid $R^3OH$. For instance, the 2-benzothiazolyl thioester may be reacted with the compound II in an inert organic solvent such as a chlorinated hydrocarbon e.g. methylene chloride, in acetone, ethyl acetate or in a mixture of such solvents with water. The 1-hydroxybenzotriazole ester can be employed by reacting the acid $R^3OH$ with 1-hydroxybenzotriazole and a carbodiimide, especially N,N'-dicyclohexylcarbodiimide or N,N'-diisopropylcarbodiimide in an inert organic solvent, preferably methylene chloride, dimethylformamide, tetrahydrofuran, acetonitrile or ethyl acetate.

The reaction of a 7-amino compound of formula II with an acid of formula $R^3OH$ or a reactive derivative thereof can conveniently be carried out at a temperature between about -40°C and +60°C, e.g. at room temperature.

Embodiment (b) of the process of the present invention involves deprotection of any of carboxy, amino and hydroxy groups of a compound of formula I. This deprotection is carried out in a manner known per se and can be illustrated as follows:

Carboxy groups:

Carboxy groups can be protected by protecting ester groups which are commonly used in the β-lactam or cephalosporin field and which are easily split off under mild conditions. Examples of such groups are described in T.W. Greene : Protective Groups in Organic Synthesis, Chapter 5, pages 152-192, John Wiley & Sons (1981). Among these carboxy protecting ester groups there may be mentioned: p-Nitrobenzyl, t-butyl, benzhydryl, allyl, lower alkanoyloxyalkyl (for example acetoxymethyl, pivaloyloxymethyl, 1-acetoxyethyl and 1-pivaloyloxyethyl), lower alkoxycarbonyloxyalkyl (for example methoxycarbonyloxymethyl, 1-ethoxycarbonyloxyethyl and 1-isopropoxycarbonyloxyethyl), lactonyl (for example phthalidyl and thiophthalidyl), lower alkoxymethyl (for example methoxymethyl) and lower alkanoylaminomethyl (for example acetamidomethyl). Other ester groups (for example benzyl, p-methoxybenzyl and cyanomethyl) can also be used. The cleavage of the protecting groups is effected in a manner known per se. For example, p-nitrobenzyl is removed by hydrolysis in the presence of sodium sulfide at about or below 0°C to room temperature in a solvent, such as dimethylformamide (aqueous); t-butyl, benzhydryl and p-methoxybenzyl are removed by reaction with trifluoroacetic acid, optionally in the presence of anisole, at about 0°C to room temperature with or without a co-solvent such as methylene chloride; allyl is removed by a palladium (O) catalyzed transallylation reaction in the presence of sodium or potassium salt of 2-ethyl hexanoic acid, see for example, J. Org. Chem. 1982, 47, 587.

Amino groups:

Amino groups are likewise protected by protecting groups commonly used in the β-lactam/cephalosporin field or peptide chemistry and easily split off under mild conditions. Examples of such groups are described in Chapter 7, pages 218-282 of "Protective Groups in Organic Synthesis" as set forth above. Among these amino protecting groups there may be mentioned:

Alkoxycarbonyl groups, e.g., t-butoxycarbonyl, etc., substituted alkoxycarbonyl groups, e.g., trichloroethoxycarbonyl etc., substituted aralkyloxycarbonyl groups, e.g., p-nitrobenzyloxycarbonyl, aralkyl groups such as trityl or benzhydryl, halogen-alkanoyl groups such as chloroacetyl, bromoacetyl, iodoacetyl or trifluoroacetyl.

Preferred protecting groups are t-butoxycarbonyl (t-BOC) and trityl.

The amino protecting groups may be cleaved off by acidic hydrolysis (e.g. the t-butoxycarbonyl or trityl group), e.g. aqueous formic acid, or by basic hydrolysis (e.g. the trifluoroacetyl group). The chloroacetyl, bromoacetyl and iodoacetyl groups are cleaved off by treatment with thiourea.

Amino-protecting groups which are cleavable by acidic hydrolysis are preferably removed with the aid of a lower alkanecarboxylic acid which may be halogenated. In particular, formic acid or trifluoroacetic acid is used. The acidic hydrolysis is generally carried out at room temperature, although it can be carried out at a slightly higher or slightly lower temperature (e.g. a temperature in the range of about 0°C to +40°C). Protecting groups which are cleavable under basic conditions are generally hydrolyzed with dilute aqueous caustic alkali at 0°C to 30°C. The chloroacetyl, bromoacetyl and iodoacetyl protecting groups can be cleaved off using thiourea in acidic, neutral or alkaline medium at about 0°C-30°C.

Hydroxy groups:

Hydroxy groups can be protected by protecting groups commonly used in the β-lactam or cephalosporin field and easily split off under mild conditions. Examples of such group are described in chapters 2 and 3, pages 10-113, of "Protective Groups in Organic Synthesis" as set forth above. Specific examples of these hydroxy protecting groups include catechol protecting groups such as diphenylmethylene, which is removed with moist trifluoroacetic acid at 0°C; phenol protecting groups such as acetyl, which is removed by hydrolysis with sodium bicarbonate in aqueous methanol at or below room temperature; and alcohol protecting groups such as dichloroacetyl, which is removed by mild basic hydrolysis at about room temperature.

In accordance with process variant (c) of the process in accordance with the invention a ketocephalosporin of formula III or a salt thereof is reacted with an O-substituted hydroxyamine of formula IV or a salt thereof. As the salts there preferably comes into consideration a mineral acid salt, e.g. the hydrochloride, or an organic sulphonate such as e.g. the p-toluenesulphonate. The salt of compound IV is preferably used in about an equimolar amount up to a slight excess. The reaction is preferably carried out in a polar organic solvent, e.g. in dimethylformamide, N-methylpyrrolidone, dimethethyl sulphoxide, acetonitrile, water or, especially, in dimethylacetamide. When the latter solvent is used, there are obtained especially high amounts of the syn form of the end product. The temperature preferably lies between 0°C and room temperature.

The manufacture of the salts and hydrates of the compounds of formula I or the hydrates of said salts in accordance with embodiment (d) of the process provided by the present invention can be carried out in a manner known per se; for example, by reacting a carboxylic acid of formula I or a salt thereof with an equivalent amount of the desired base, conveniently in a solvent such as water or an organic solvent (e.g. ethanol, methanol, acetone and the like). Correspondingly, salt formation is brough about by the addition of an organic or inorganic salt. The temperature at which the salt formation is carried out is not critical. The salt formation is generally carried out at room temperature, but it can be carried out at a temperature slightly above or below room temperature, for example in the range of 0°C to +50°C.

The manufacture of the hydrates usually takes place automatically in the course of the manufacturing process or as a result of the hygroscopic properties of an initially anhydrous product. For the controlled manufacture of a hydrate, a completely or partially anhydrous carboxylic acid of formula I or salt thereof can be exposed to a moist atmosphere (e.g. at about +10°C to +40°C).

The following reaction schemes illustrate the above process for producing the novel compounds of the present invention:

## REACTION SCHEME 1

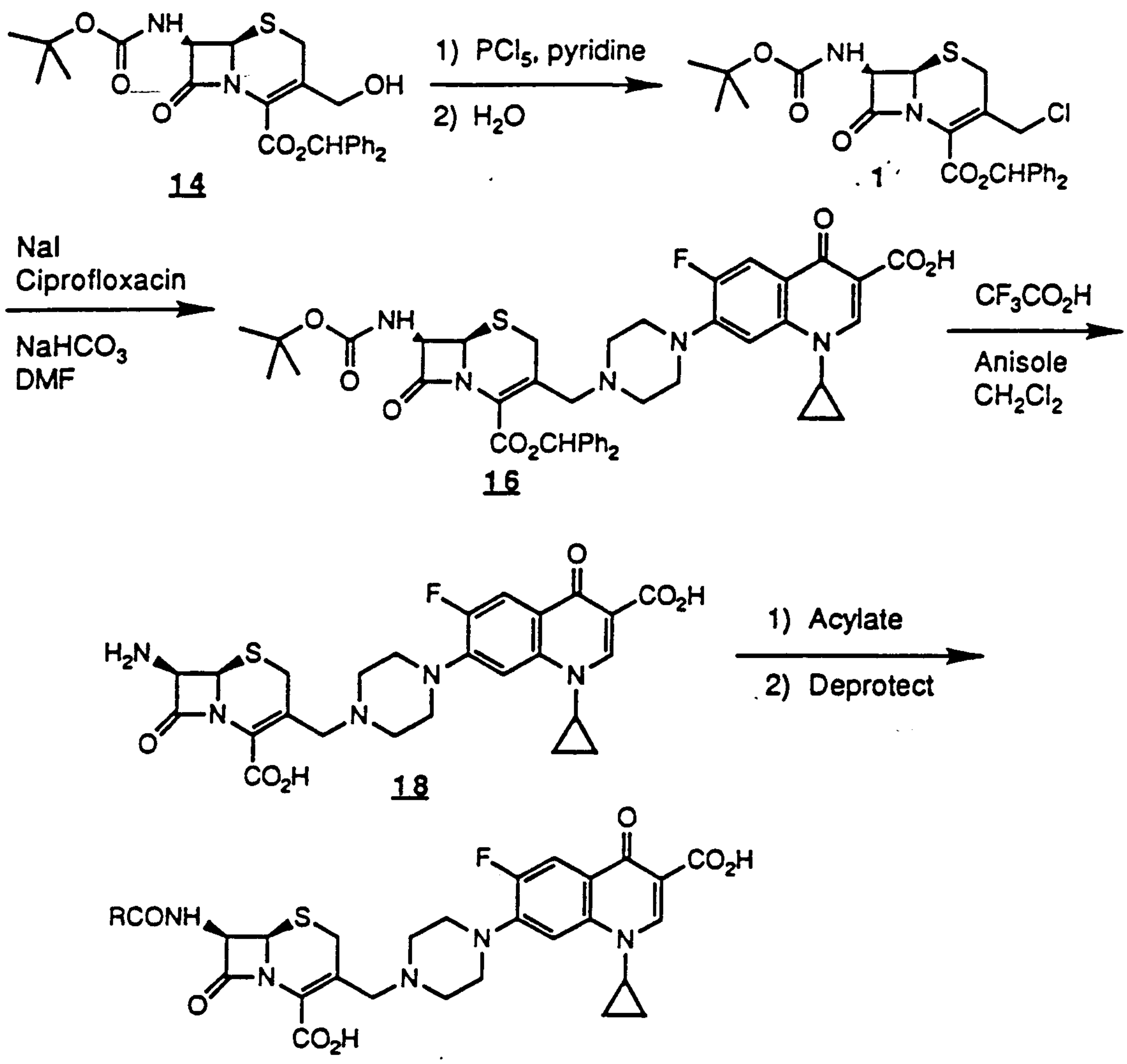


Compound A; R=

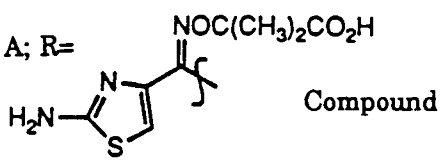

Compound B; R=

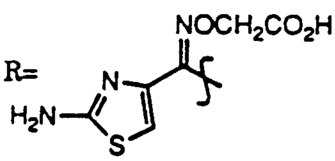

REACTION SCHEME 2

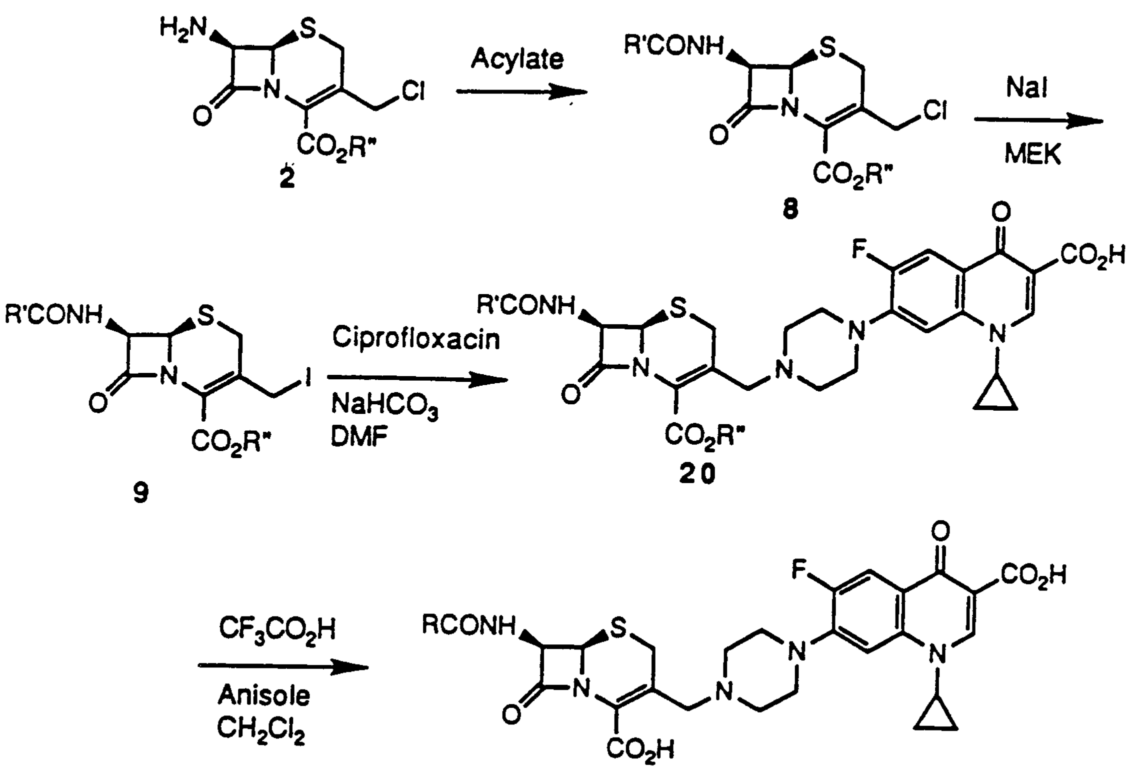


2 8 9 20

Compound C; R=R'= 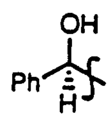

Compound D; R= 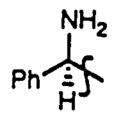 , R' = 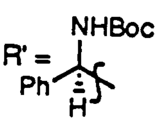

Compound E; R=R'= 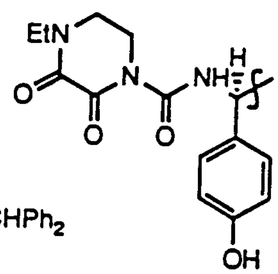

Compound F; R= 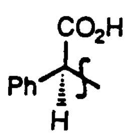 R' = 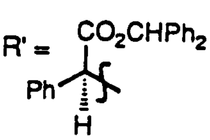

## REACTION SCHEME 3

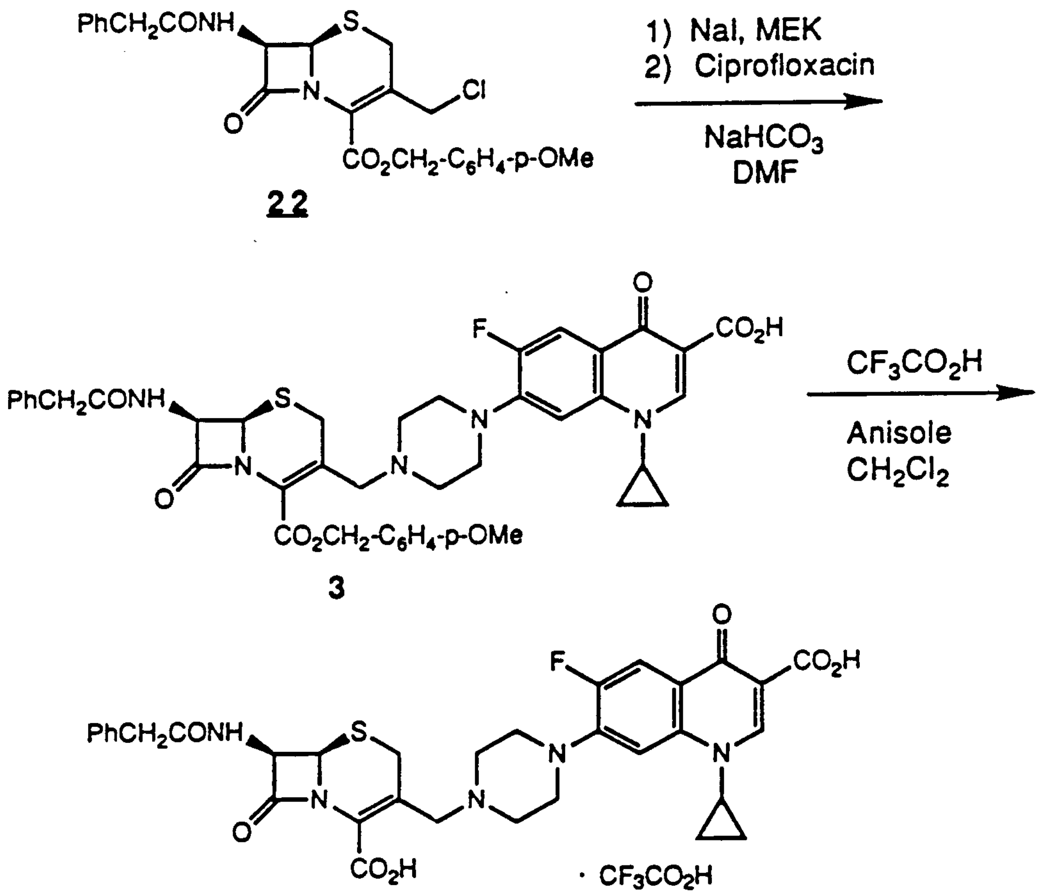


22

3

26

REACTION SCHEME 4

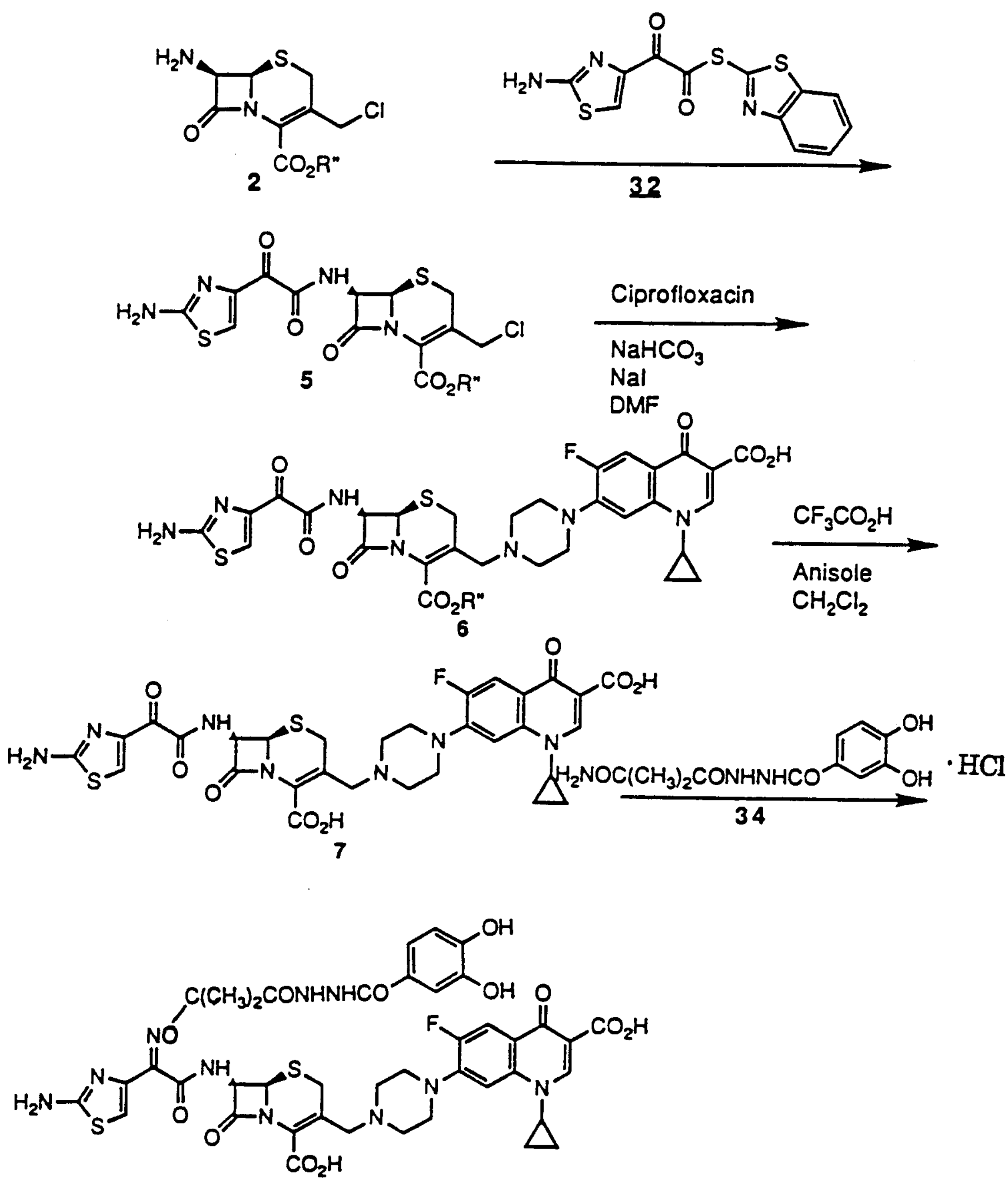


2, 32, 5, 6, 7, 34

Compound G

In the above Reaction Schemes 1-4 those symbols not explained in the schemes or otherwise not readily apparent have meanings as follows;

| | | |
|---|---|---|
| Ph | = | phenyl |
| DMF | = | dimethylformamide |
| R" | = | benzhydryl, p-methoxybenzyl |
| MEK | = | methylethyl ketone |
| Boc | = | t-butoxycarbonyl |
| Et | = | ethyl |
| Me | = | methyl |

In the Examples below, all mixtures of solvents are on a volume per volume basis unless otherwise stated. All other solutions are on a weight per weight basis unless otherwise stated. All temperatures are in degrees Celsius unless otherwise stated.

Example 1

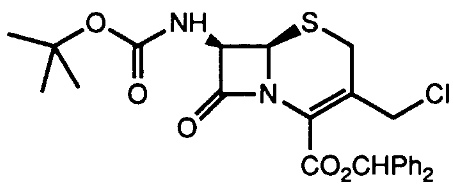

(6R-trans)-3-(Chloromethyl)-7-[[(1,1-dimethylethoxy)carbonyl]amino]-8-oxo-5-thia-1-azabicyclo[4.2.0]oct-2-ene-2-carboxylic acid diphenylmethyl ester (1)

A solution of 9.93 g (0.02 mol) of (6R-trans)-3-(hydroxymethyl)-7-[[(1,1-dimethylethoxy)carbonyl]amino]-8-oxo-5-thia-1-azabicyclo[4.2.0]oct-2-ene-2-carboxylic acid diphenylmethyl ester (14) in 200 ml of methylene chloride was cooled to -40 °C. In one portion, 5.00 g (0.024 mol) of phosphorous pentachloride was added, followed within 1 to 2 minutes by 2.00 ml (0.025 mol) of pyridine. The reaction was stirred at -30 to -40 °C for 1.5 hours, and then poured into a cold mixture of 250 ml of water and 100 ml of methylene chloride. The organic phase was washed with water and aqueous sodium bicarbonate, dried over $Na_2SO_4$, and concentrated under reduced pressure to obtain a quantitative yield of the title compound.

Example 2

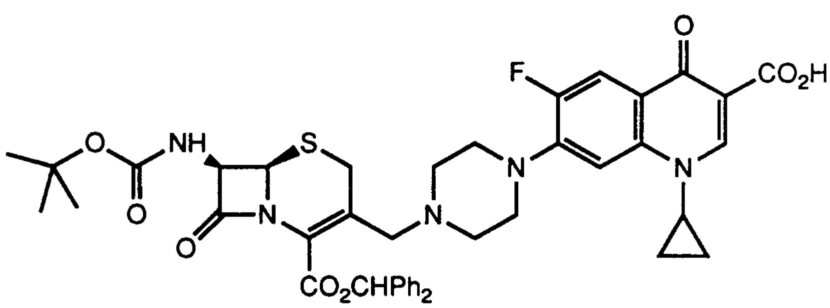

(6R-trans)-3-[[4-(3-Carboxy-1-cyclopropyl-6-fluoro-1,4-dihydro-4-oxo-7-quinolinyl)-1-piperazinyl]methyl]-7-[-[1,1-dimethylethoxy)carbonyl]amino]-8-oxo-5-thia-1-azabicyclo[4.2.0]oct-2-ene-2-carboxylic acid diphenylmethyl ester (16)

A mixture of 2.58 g (0.005 mol) of the product from Example 1 (1), 0.75 g (0.005 mol) of sodium iodide, and 100 ml of DMF was stirred for 30 minutes; 1.66 g (0.005 mol) of ciprofloxacin and 0.50 g (0.006 mol) of sodium bicarbonate were then added, and stirring continued overnight. The insoluble portion was removed by filtration, and the residue taken up in a mixture of 200 ml of ethyl acetate, 200 ml of methylene chloride, and 100 ml of water. The aqueous phase was separated and extracted with 100 ml of 1:1 ethyl acetate:methylene chloride. The combined organic extracts were dried over $Na_2SO_4$ and concentrated under reduced pressure. The residue was triturated with ether to obtain 1.86 g of crude product as a solid. Chromatographic purification of 1.23 g of this material using 18 g of 70 - 230 mesh silica gel and 5% methanol/chloroform as eluent gave 0.99 g (37%) of the title compound. IR (KBr) 1785, 1722, 1628 $cm^{-1}$; MS m/z 810 $(M + H)^+$.

Example 3

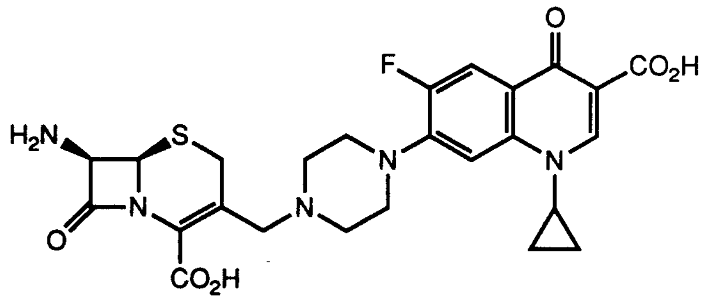

(6R-trans)-7-Amino-3-[[4-(3-carboxy-1-cyclopropyl-6-fluoro-1,4-dihydro-4-oxo-7-quinolinyl)-1-piperazinyl]-methyl]-8-oxo-5-thia-1-azabicyclo[4.2.0]oct-2-ene-2-carboxylic acid trifluoracetic acid salt (18)

A solution of 1.32 g (0.00163 mol) of the above compound (16) in a mixture of 53 ml of trifluoroacetic acid, 53 ml of methylene chloride, and 5.3 ml of anisole was kept at 0 to 5 ° C for 16 hours. The mixture was concentrated to dryness under reduced pressure. Trituration of the residue with ether provided 1.20 g (95%) of the title compound as a solid. IR (KBr) 1789, 1712 - 1677, 1630 $cm^{-1}$; MS m/z 544 $(M + H)^+$.

Example 4

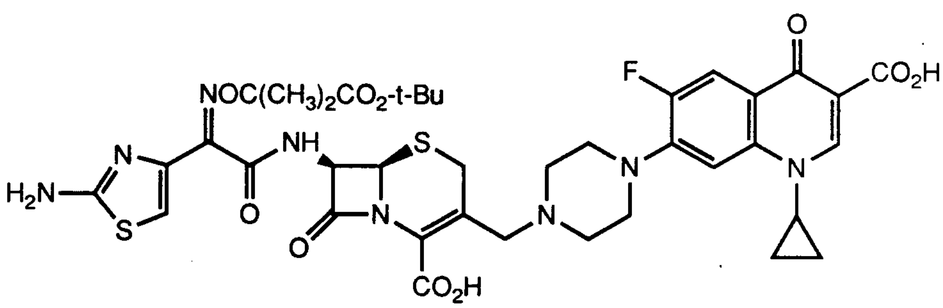

[6R-[6α,7β(Z)]-7[[[(2-Amino-4-thiazolyl)[[1-(1,1-dimethylethoxy)carbonyl]-1-methylethoxy]imino]acetyl]-amino]-3-[[4-(3-carboxy-1-cyclopropyl-6-fluoro-1,4-dihydro-4-oxo-7-quinolinyl)-1-piperazinyl]methyl-8-oxo-4-thia-1-azabicyclo[4.2.0]oct-2-ene-2-carboxylic acid (18a)

A mixture of 0.537 g (0.786 mmol) of the product from Example 3 (18), 15 ml of tetrahydrofuran (THF), 0.317 g (3.78 mmol) of sodium bicarbonate, and 15 ml of water was stirred at 0 to 5 ° C for 20 minutes. A solution of 0.768 g (1.61 mmol) of (Z)-2-[[[1-(2-amino-4-thiazolyl)-2-benzothiazol-2-ylthio)-2-oxoethyl]imino]-oxy]-2-methylpropanoic acid 1,1-dimethylethyl ester in 10 ml of THF was then added, and stirring continued for 15 minutes at 0 to 5 ° C. The cooling bath was then removed, and the mixture stirred overnight at room temperature. The THF was evaporated under reduced pressure, and the residual aqueous solution was washed with ethyl acetate. The pH was adjusted to 5.5 by adding 2N aqueous HCl, to precipitate a solid. After filtering, washing with water and ethyl acetate, and drying under reduced pressure, 0.424 g (63%) of product was obtained.

Example 5

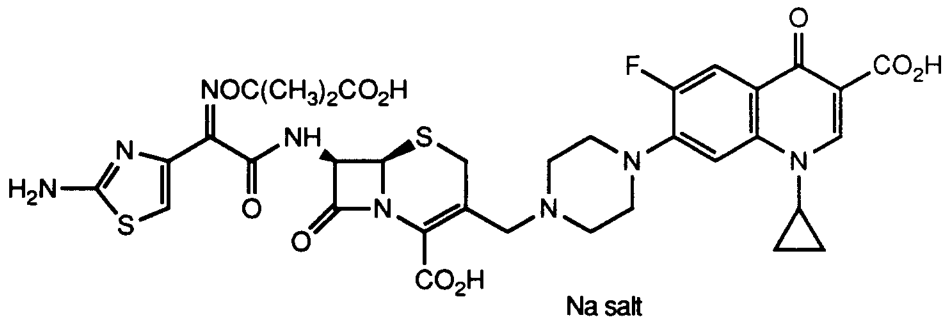

Na salt

[6R-[6α,7β(Z)]]-7-[[(2-Amino-4-thiazolyl)[(1-carboxy-1-methylethoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]-oct-2-ene-2-carboxylic acid sodium salt (Compound A)

A solution of 0.472 g (0.553 mmol) of the above intermediate (18a) in 19 ml of trifluoroacetic acid, 1.9 ml of anisole, and 19 ml of methylene chloride was kept at 0 to 5°C for 16 hours. The mixture was concentrated to dryness under reduced pressure. Methylene chloride was added, and the evaporation was repeated. The residue was triturated with ether to obtain solid product in the form of a trifluoroacetic acid salt. This was dissolved in aqueous sodium bicarbonate at pH 7 and purified as the sodium salt on a column of 20 g of Waters $C_{18}$ silica, eluting with an aqueous acetonitrile gradient of up to 30% acetonitrile. The appropriate fractions were combined, concentrated under reduced pressure and freeze-dried to obtain 0.363 g (78%) of Compound A. IR (KBr) 1762, 1658, 1628 $cm^{-1}$; MS m/z 843 $(M + H)^+$.

Example 6

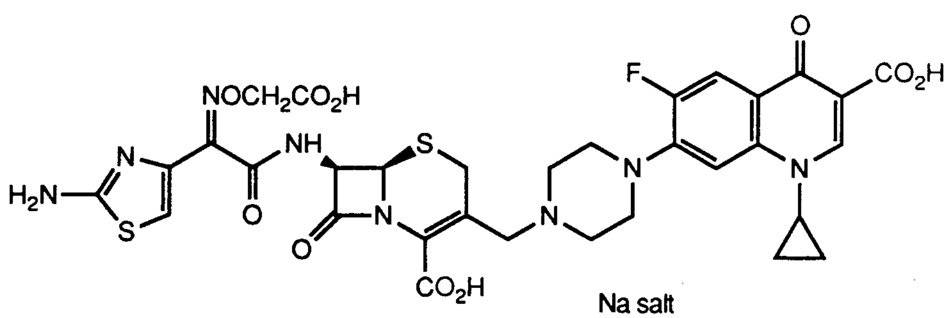

Na salt

[6R-[6α,7β(Z)]]-7-[[(2-Amino-4-thiazolyl)[(carboxy-methoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]-oct-2-ene-2-carboxylic acid sodium salt (Compound B)

Using procedures similar to those described above for the preparation of Compound B, intermediate (18) was acylated in 74% yield with (Z)-[[[1-(2-amino-4-thiazolyl)-2-(benzothiazol-2-ylthio)-2-oxoethyl]imino]-oxy]acetic acid 1,1-dimethylethyl ester and then deprotected with trifluoroacetic acid - anisole in methylene chloride. After purification as the sodium salt by $C_{18}$ reverse phase chromatography, Compound D was obtained in 71% yield. IR (KBr) 3410, 1760, 1622 $cm^{-1}$; MS m/z 815 $(M + H)^+$.

Example 7

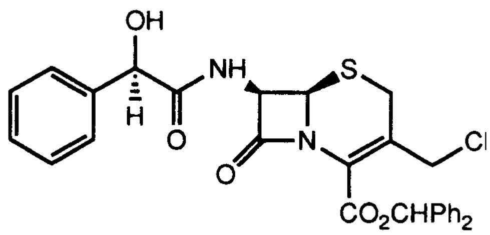

[6R-[6α,7β(R)]]-3-(Chloromethyl)-7-[(hydroxyphenyl-acetyl)amino]-8-oxo-5-thia-1-azabicyclo[4.2.0]oct-2-ene-2-carboxylic acid diphenylmethyl ester (8)

A mixture of 1.26 g (3.05 mmol) of (6R-trans)-7-amino-3-chloromethyl-8-oxo-5-thia-1-azabicyclo[4.2.0]-oct-2-ene-2-carboxylic acid diphenylmethyl ester (2), 0.464 g (3.05 mmol) of R-mandelic acid, and 0.412 g (3.05 mmol) of 1-hydroxybenzotriazole in 30 ml of methylene chloride was stirred; 0.628 g (3.05 mmol) of 1,3-dicyclohexylcarbodiimidewas added, and the mixture stirred for 16 hours. The insoluble portion was removed by filtration. The filtrate was washed with 0.1 molar phosphoric acid, followed by water. The methylene chloride solution was dried over $Na_2SO_4$ and concentrated to dryness under reduced pressure. The residue solidified on trituration with ether. After twice more triturating with 10 ml portions of acetonitrile, 0.881 g (53%) of the titled product was obtained.

Example 8

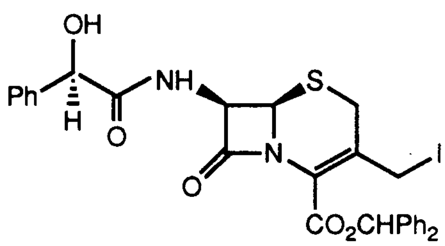

[6R-[6α,7β(R)]]-7-[(Hydroxyphenylacetyl)amino]-3-(iodomethyl)-8-oxo-5-thia-1-azabicyclo[4.2.0]oct-2-ene-2-carboxylic acid diphenylmethyl ester (9)

A mixture of 0.20 g (0.364 mmol) of the above chloro compound from Example 7 (8), 0.273 g (1.82 mmol) of sodium iodide and 6 ml of methyl ethyl ketone was stirred at room temperature for two hours. The solvent was evaporated under reduced pressure and the residue taken up in ethyl acetate. The organic solution was washed with cold 5% aqueous sodium thiosulfate and brine, dried over $Na_2SO_4$, and concentrated to dryness under reduced pressure to obtain 0.217 g (93%) of the titled product.

Example 9

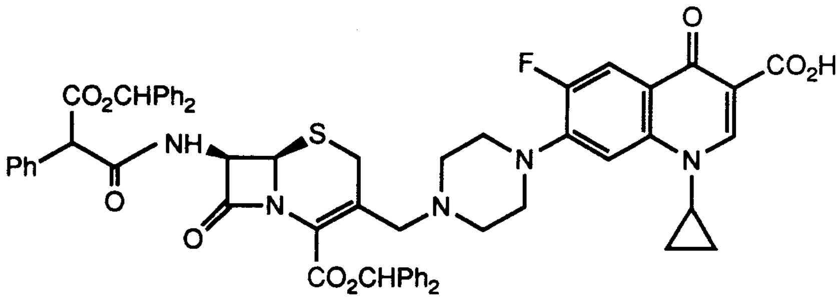

[6R-[6α,7β(R)]]-3-[[4-(3-Carboxy-1-cyclopropyl-6-fluoro-1,4-dihydro-7-quinolinyl)-1-piperazinyl]methyl]-7-[-(hydroxyphenylacetyl)amino]-8-oxo-5-thia-1-azabicyclo[4.2.0]oct-2-ene-2-carboxylic acid diphenylmethyl ester (20)

A mixture of 0.217 g (0.338 mmol) of the iodide prepared in Example 8, 0.112 g (0.338 mmol) of ciprofloxacin, 28.4 mg (0.338 mmol) of sodium bicarbonate, and 7 ml of DMF was stirred for three hours. The mixture was concentrated under reduced pressure. Water and a 1:1 mixture of ethyl acetate:methylene chloride were added to the residue. The organic phase was dried over $Na_2SO_4$ and concentrated under reduced pressure to give 0.238 g (83%) of title product.

Example 10

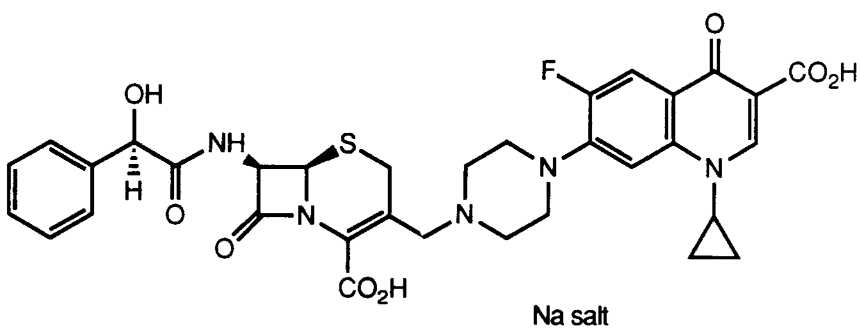

Na salt

[6R-[6α,7β(R)]]-3-[[4-(3-Carboxy-1-cyclopropyl-6-fluoro-1,4-dihydro-4-oxo-7-quinolinyl)-1-piperazinyl]methyl]-7-[(hydroxyphenylacetyl)amino]-8-oxo-5-thia-1-azabicyclo[4.2.0]oct-2-ene-2-carboxylic acid sodium salt (Compound C)

A solution of 90.5 mg of the product of Example 9 in 3.6 ml trifluoroacetic acid, 3.6 ml of methylene chloride, and 0.36 ml of anisole was kept at 0 °C for 15 minutes. The mixture was concentrated to dryness under reduced pressure, and the residue triturated with ether to obtain 67.0 mg of solid trifluoroacetic acid salt. This solid was dissolved in aqueous sodium bicarbonate at pH 8, and purified by reverse phase chromatography on 2 g of $C_{18}$ silica, eluting with an acetonitrile - water gradient of 0 to 30% acetonitrile. The appropriate fractions were combined, concentrated under reduced pressure, and freeze dried to yield 40.6 mg (54%) of product. IR (KBr) 1758, 1665, 1628 $cm^{-1}$; MS m/z 700 $(M + H)^+$, 722 $(M + Na)^+$.

Example 11

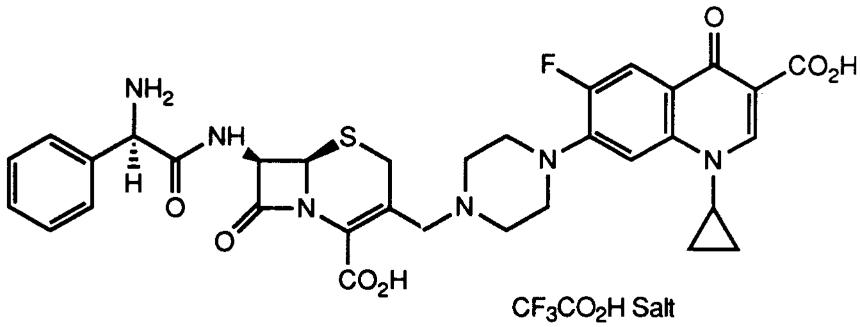

$CF_3CO_2H$ Salt

[6R-[6α,7β(R)]]-7-[(Aminophenylacetyl)amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid trifluoroacetic acid salt (Compound D)

From the starting materials N-t-Boc protected R-phenylglycine and (6R-trans)-7-amino-3-chloromethyl-8-oxo-5-thia-1-azabicyclo[4.2.0]oct-2-ene-2-carboxylic acid diphenylmethyl ester (2), using procedures similar to those set forth in Examples 7 through 10, Compound D was prepared.

Example 12

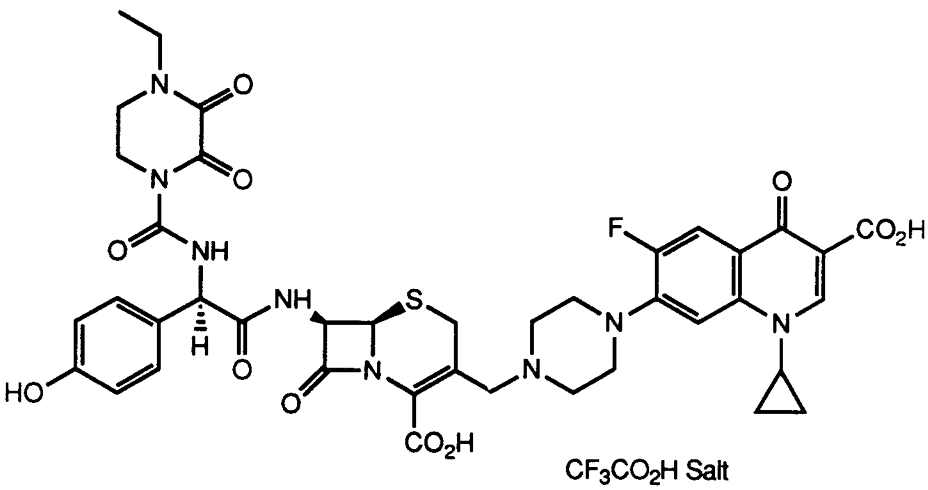

$CF_3CO_2H$ Salt

[6R-[6α,7β(R)]]-3-[[4-(3-Carboxy-1-cyclopropyl-6-fluoro-1,4-dihydro-4-oxo-7-quinolinyl)-1-piperazinyl]methyl]-7-[[[[(4-ethyl-2,3-dioxo-1-piperazinyl)carbonyl]amino](4-hydroxyphenyl)acetyl]amino]-8-oxo-5-thia-1-azabicyclo[4.2.0]oct-2-ene-2-carboxylic acid trfluoroacetic acid salt (Compound E)

From the starting materials (R)-[[(4-ethyl-2,3-dioxo-1-piperazinyl)carbonyl]amino](4-hydroxyphenyl)acetic acid and (6R-trans)-7-amino-3-chloromethyl-8-oxo-5-thia-1-azabicyclo[4.2.0]oct-2-ene-2-carboxylic acid diphenylmethyl ester (2), using procedures similar to those set forth in Examples 7 through 10, Compound E was prepared, and characterized as a trifluoroacetic acid salt. IR (KBr) 1785, 1715, 1680 $cm^{-1}$; MS m/z 883 $(M + H)^+$.

Example 13

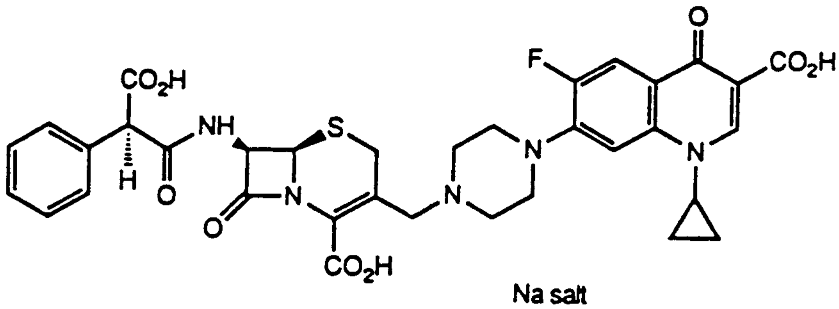

Na salt

(6R-trans)-3-[[4-(3-Carboxy-1-cyclopropyl-6-fluoro-1,4-dihydro-4-oxo-7-quinolinyl)-1-piperazinyl]methyl]-7-[-(carboxyphenylacetyl)amino]-8-oxo-5-thia-1-azabicyclo[4.2.0]oct-2-ene-2-carboxylic acid sodium salt (Compound F)

Using as the starting acid phenylmalonic acid monodiphenylmethyl ester, and using procedures similar to those set forth in Examples 7 through 10 Compound F was prepared. MS m/z 750 (M + H)$^+$.

Example 14

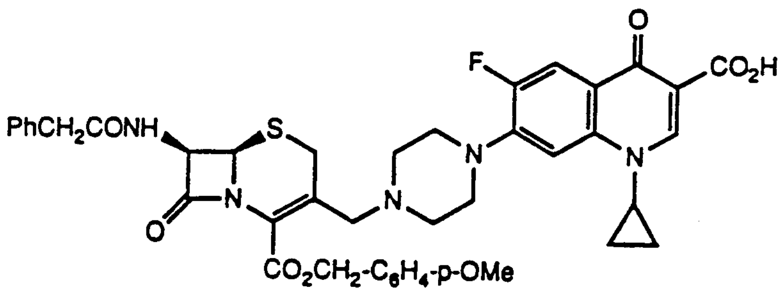

(6R-trans)-3-[[4-(3-Carboxy-1-cyclopropyl-6-fluoro-1,4-dihydro-4-oxo-7-quinolinyl]-1-piperazinyl]methyl]-8-oxo-7-[(phenylacetyl)amino]-5-thia-1-azabicyclo[4.2.0]oct-2-ene-2-carboxylic acid 4-methoxybenzyl ester (3)

A mixture of 0.49 g (1 mmol) of (6R-trans)-3-(chloromethyl)-8-oxo-7-[(phenylacetyl)amino]-5-thia-1-azabicyclo[4.2.0]oct-2-ene-2-carboxylic acid 4-methoxybenzyl ester (22), 0.75 g (5 mmol) of sodium iodide, and 12 ml of methyl ethyl ketone was stirred for two hours. The solvent was evaporated under reduced pressure, and the residue was taken up in 25 ml of methylene chloride and 15 ml of water. The organic phase was separated and washed with 15 ml of 5% aqueous sodium thiosulfate solution, followed by 15 ml of water. The organic phase was dried over $Na_2SO_4$ and concentrated under reduced pressure. A mixture of this residue with 332 mg (1 mmol) of ciprofloxacin, 101 mg (1.2 mmol) of sodium bicarbonate, and 20 ml of DMF was stirred for 19 hours. The mixture was filtered to remove a small amount of undissolved solid, and the solution was concentrated to dryness under reduced pressure. The residue was taken up in 40 ml of methylene chloride and 25 ml of water. The organic phase was separated, dried over $Na_2SO_4$, and concentrated under reduced pressure. On trituration with 15 ml of ether, the residue gradually solidified to give 402 mg (52.2%) of the titled product.

Example 15

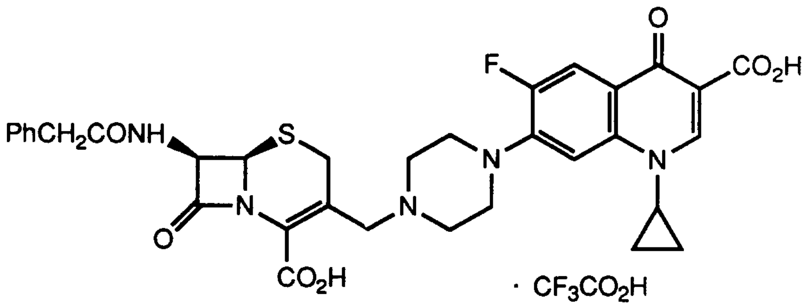

(6R-trans)-3-[[4-(3-Carboxy-1-cyclopropyl-6-fluoro-1,4-dihydro-4-oxo)-7-quinolinyl)-1-piperazinyl]methyl]-8-oxo-7-[(phenylacetyl)amino]-5-thia-1-azabicyclo[4.2.0]oct-2-ene-2-carboxylic acid trifluoroacetic acid salt (26)

A solution of 0.34 g (0.44 mmol) of the product from Example 14 (3) in 7 ml of methylene chloride and 0.7 ml of anisole was chilled in ice; 7 ml of trifluoroacetic acid was added, and the solution was kept at 0 ° C for one hour. The mixture was then concentrated to dryness under reduced pressure. Methylene chloride was added to the residue, and again the mixture was concentrated to dryness. The residue was triturated with 50 ml of ether. The resulting solid was filtered, washed with ether, and dried under reduced pressure to obtain 0.28 g (81.7%) of the titled product. IR (KBr) 1782, 1718, 1668, 1628 $cm^{-1}$; MS m/z 662 $(M + H)^+$.

Example 16

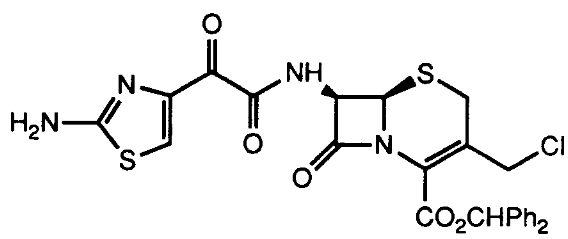

(6R-trans)-7-[[(2-Amino-4-thiazolyl)(oxo)acetyl]amino]-3-(chloromethyl)-8-oxo-5-thia-1-azabicyclo[4.2.0]oct-2-ene-2-carboxylic acid diphenylmethyl ester (5)

A mixture of 207 mg (0.5 mmol) of (6R-trans)-7-amino-3-chloromethyl-8-oxo-5-thia-1-azabicyclo[4.2.0]-oct-2-ene-2- carboxylic acid diphenylmethylthyl ester (2) and 208 mg (0.65 mmol) of 2-amino-4-thiazolethioglyoxylic acid S-(2-benzothiazolyl) ester in 30 ml of THF was stirred at room temperature for three hours. The solvent was evaporated under reduced pressure, and the residue purified by flash chromatography on a column of 230 - 400 mesh silica, with a 2:3 mixture of ethyl acetate and methylene chloride, to obtain 225 mg (79%) of the titled product.

Example 17

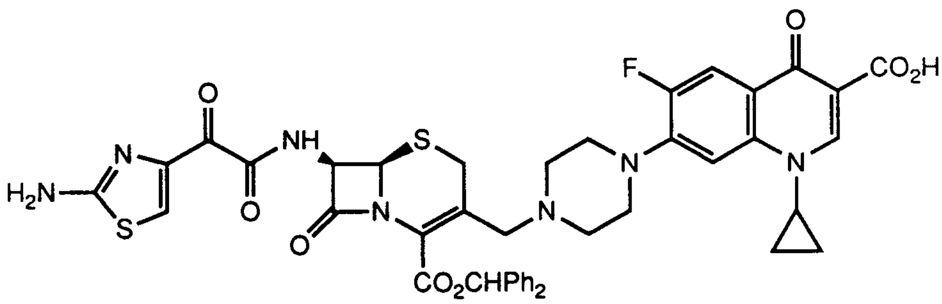

(6R-trans)-7-[[(2-Amino-4-thiazolyl)(oxo)acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo-[4.2.0]oct-2-ene-2-carboxylic acid diphenylmethyl ester (6)

A mixture of 225 mg (0.396 mmol) of the product from Example 16, 131 mg (0.396 mmol) of ciprofloxacin, 40 mg (0.475 mmol) of sodium bicarbonate, 59 mg (0.396 mmol) of sodium iodide, and 4 ml of DMF was stirred for six hours. A small amount of undissolved solid was removed by filtration. The filtrate was concentrated to dryness under reduced pressure. The residue was triturated with THF, the insoluble portion filtered and discarded, and the filtrate evaporated under reduced pressure. The residue was triturated was cold ethanol to provide 280 mg (82%) of the titled product.

Example 18

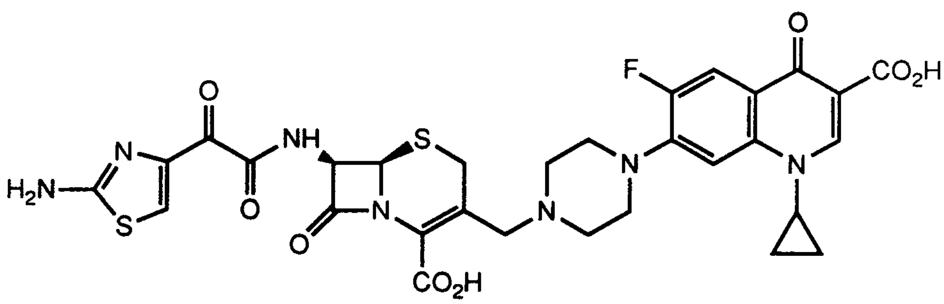

(6R-trans)-7-[[(2-Amino-4-thiazolyl)(oxo)acetyl]-amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid trifluoroacetic acid salt (7)

A suspension of 280 mg (0.32 mmol) of the product of Example 17 (6) in 6 ml of methylene chloride and 0.8 ml of anisole was stirred and cooled in ice; 5.5 ml of trifluoroacetic acid was added dropwise over a period of five minutes, and the mixture was stirred at 0 °C for 30 minutes. The solution was concentrated to dryness under reduced pressure. The residue was triturated repeatedly with portions of petroleum ether. The residual oil was then triturated with a mixture of 1 ml of methylene chloride and 5 ml of ethyl acetate to yield 215 mg of solid. After further washing with methanol and with acetone, 130 mg (50%) of the title product was obtained.

Example 19

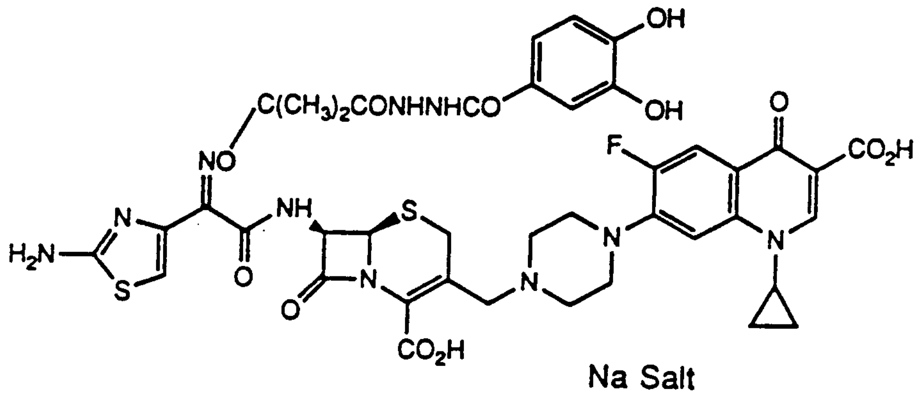

Na Salt

[6R-[6α,7β(Z)]]-7-[[[(2-Amino-4-thiazolyl)[1-[[(3,4-dihydroxybenzoyl)hydrazino]carbonyl]-1-methylethoxy]-imino]acetyl]amino]-3-[[4-(3-carboxy-1-cyclopropyl-6-fluoro-1,4-dihydro-4-oxo-7-quinolinyl)-1-piperazinyl]-methyl]-8-oxo-5-thia-1-azabicyclo[4.2.0]oct-2-ene-2-carboxylic acid sodium salt (Compound G)

A mixture of 130 mg (0.16 mmol) of the product from Example 18 (7) and 90 mg (0.267 mmol) of 3,4-dihydroxybenzoic acid 2-[2-(aminooxy)-2-methyl-1-oxopropyl]hydrazide hydrochloride (34) in 1.2 ml of N,N-dimethylacetamide was stirred at 0 °C for 64 hours. The mixture was concentrated to dryness under reduced pressure, and the residue stirred with 12 ml of water at 0 °C for one hour. The resulting solid was filtered, and then dissolved in 10 ml of water by added sodium bicarbonate to pH 7.4. The aqueous solution was washed with ethyl acetate and methylene chloride, and the product was precipitated by adding 2N aqueous HCl to pH 6.5. The precipitate was centrifuged, washed with water, and centrifuged again to obtain, after drying, 90 mg (60%) of solid.

This material was stirred with 2.35 ml of propylene glycol and 0.85 ml of acetone; 0.06 ml (0.12 mol) of 2N sodium 2-ethylhexanoate in acetone was added. On continued stirring complete solution occurred. Over a period of one hour, 20 ml of acetone was added to precipitate the sodium salt of the product. This precipitate was triturated with 5 ml of acetone, filtered, and dried under reduced pressure to obtain 40 mg of the titled product. MS m/z 971 $(M + H)^+$.

Example 20

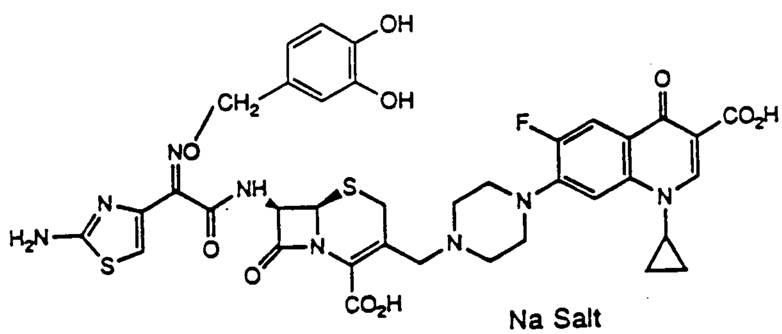

Na Salt

[6R[6α,7β(Z)]]-7-[[(2-Amino-4-thiazolyl)[(3,4-dihydroxybenzyloxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]-oct-2-ene-2-carboxylic acid sodium salt (Compound H)

By replacing 3,4-dihydroxybenzoic acid 2-[2-(aminooxy)-2-methyl-1-oxopropyl]hydrazide hydrochloride in Example 19 with O-(3,4-Dihydroxybenzyl)hydroxylamine hydrochloride the above title compound H is obtained.

The following Example illustrates pharmaceutical preparations containing the cephalosporin derivatives provided by the present invention:

Example A

Production of dry ampoules for intramuscular administration:

A lyophilisate of 1 g of active ingredient is prepared in the usual manner and filled into an ampoule. The sterile water ampoule contains 10% propylene glycol. Prior to the administration, the lyophilisate is treated with 2,5 ml of a 2% aqueous lidocaine hydrochloride solution.

As active ingredient can be used one of the end products prepared according to the above Examples.

**Claims**

**1.** Cephalosporin derivatives of the general formula

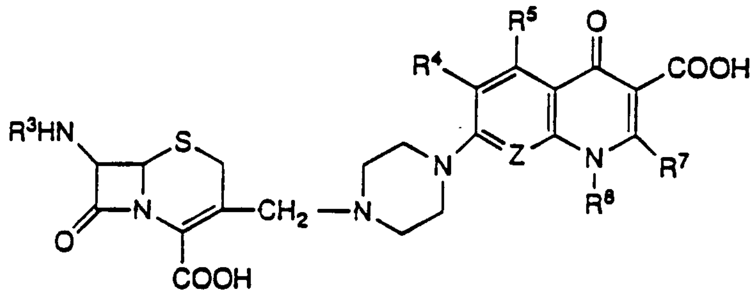

I

in which $R^3$ is a group of the formula

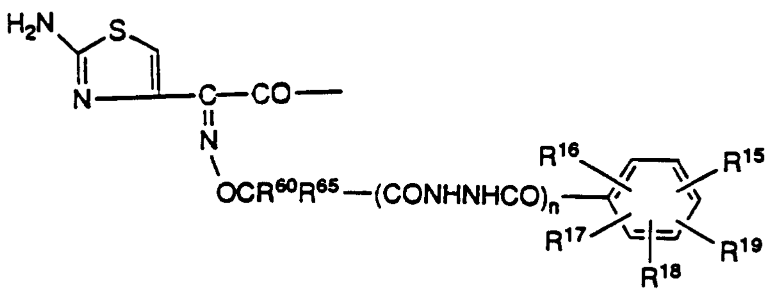

(a)

$R^{60}$ and $R^{65}$ are hydrogen or lower alkyl, $R^{15}$-$R^{19}$ are each independently hydrogen, halogen or hydroxy, n is zero or 1, $R^4$ and $R^5$ are each hydrogen, halogen, lower alkyl, lower alkoxy or amino, $R^7$ is hydrogen or lower alkyl and $R^8$ is lower alkyl, halo- lower alkyl, $C_3$-$C_7$ cycloalkyl or mono-, di- or trihalophenyl, and Z is N or C-$R^9$, where $R^9$ is hydrogen, halogen, lower alkyl or lower alkoxy; and, when $R^4$ is fluoro, $R^5$ and $R^7$ are hydrogen, $R^8$ is cyclopropyl and Z is C-H, $R^3$ can also be one of the groups

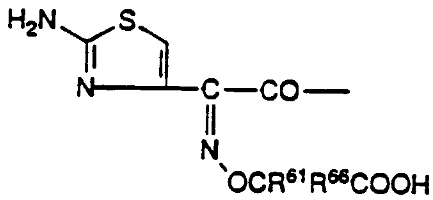

(b)

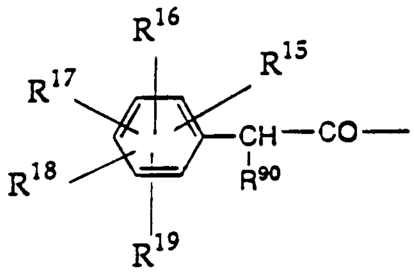

(c)

in which $R^{61}$ and $R^{66}$ are hydrogen or methyl, $R^{15}$-$R^{19}$ are as above and $R^{90}$ is hydrogen, hydroxy, carboxy, amino or [[4-(lower alkyl)-2,3-dioxo-1-piperazinyl]carbonyl]amino;
pharmaceutically acceptable acid or base addition salts thereof, hydrates of compounds of formula I and hydrates of such salts.

**2.** Compounds of claim 1 wherein Z is C-$R^9$ where $R^9$ is hydrogen or halogen.

**3.** Compounds of claim 1 or 2 wherein $R^4$ is fluoro, $R^5$ and $R^7$ are hydrogen, $R^8$ is cyclopropyl and Z is C-H.

**4.** Compounds of any one of claims 1-3 wherein $R^3$ is selected from the group consisting of

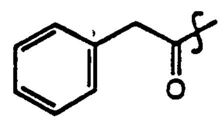

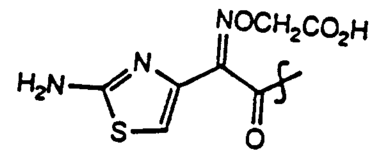

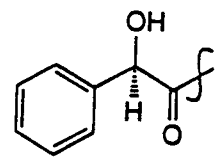

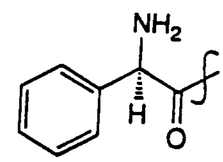

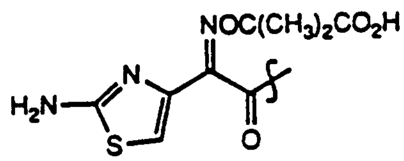

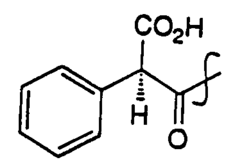

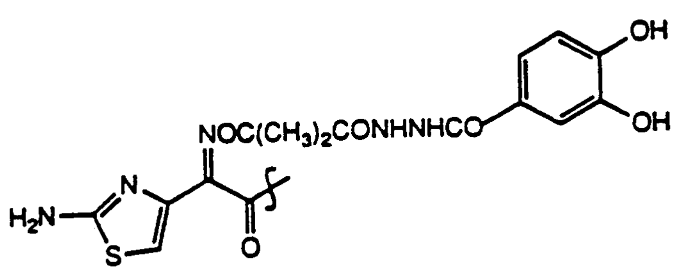

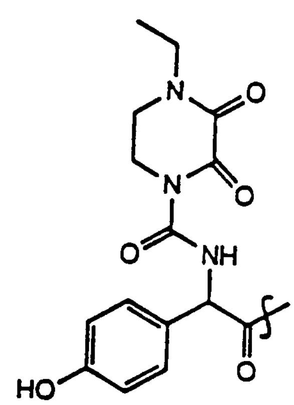

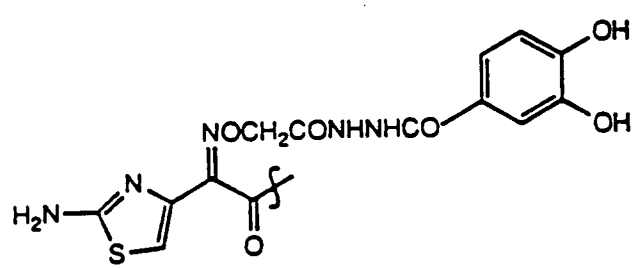

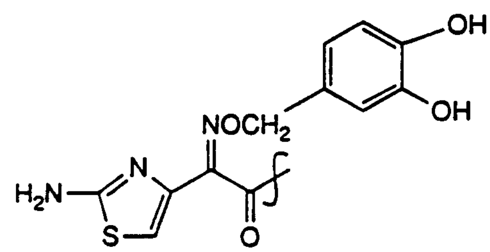

**5.** [6R-[6α,7β(Z)]]-7-[[[(2-Amino-4-thiazolyl)[1-[[(3,4-dihydroxybenzoyl)hydrazino]carbonyl]-1-methylethoxy]imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid and pharmaceutically acceptable acid or base addition salts thereof.

6. [6R-[6α,7β(Z)]]-7-[[(2-Amino-4-thiazolyl)[(1-carboxy-1-methylethoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid and pharmaceutically acceptable acid or base addition salts thereof.

7. [6R-[6α,7β(Z)]]-7-[[(2-Amino-4-thiazolyl)[(carboxymethoxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo-[4.2.0]oct-2-ene-2-carboxylic acid and pharmaceutically acceptable acid or base addition salts thereof.

8. [6R-[6α,7β(R)]]-3-[[4-(3-Carboxy-1-cyclopropyl-6-fluoro-1,4-dihydro-4-oxo-7-quinolinyl)-1-piperazinyl]-methyl]-7-[(hydroxyphenylacetyl)amino]-8-oxo-5-thia-1-azabicyclo[4.2.0]oct-2-ene-2-carboxylic acid and pharmaceutically acceptable acid or base addition salts thereof.

9. [6R-[6α,7β(R)]]-7-[(Aminophenylacetyl)amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo[4.2.0]oct-2-ene-2-carboxylic acid and pharmaceutically acceptable acid or base addition salts thereof.

10. [6R-[6α,7β(R)]]-3-[[4-(3-Carboxy-1-cyclopropyl-6-fluoro-1,4-dihydro-4-oxo-7-quinolinyl)-1-piperazinyl]-methyl]-7-[[[[(4-ethyl-2,3-dioxo-1-piperazinyl)carbonyl]amino](4-hydroxyphenyl)-acetyl]amino]-8-oxo-5-thia-1-azabicyclo[4.2.0]oct-2-ene-2-carboxylic acid and pharmaceutically acceptable acid or base addition salts thereof.

11. (6R-trans)-3-[[4-(3-Carboxy-1-cyclopropyl-6-fluoro-1,4-dihydro-4-oxo-7-quinolinyl)-1-piperazinyl]methyl]-7-[(carboxyphenylacetyl)amino]-8-oxo-5-thia-1-azabicyclo[4.2.0]oct-2-ene-2-carboxylic acid and pharmaceutically acceptable acid or base addition salts thereof.

12. (6R-trans)-3-[[4-(3-Carboxy-1-cyclopropyl-6-fluoro-1,4-dihydro-4-oxo)-7-quinolinyl)-1-piperazinyl]methyl]-8-oxo-7-[(phenylacetyl)amino]-5-thia-1-azabicyclo[4.2.0]oct-2-ene-2-carboxylic acid and pharmaceutically acceptable acid or base addition salts thereof.

13. [6R-(6α,7β(Z)]]-7-[[(2-Amino-4-thiazolyl)[(3,4-dihydroxybenzyloxy)imino]acetyl]amino]-3-[[4-(3-carboxy-1-cyclopropyl-6-fluoro-1,4-dihydro-4-oxo-7-quinolinyl)-1-piperazinyl]methyl]-8-oxo-5-thia-1-azabicyclo-[4.2.0]oct-2-ene-2-carboxylic acid and pharmaceutically acceptable acid or base addition salts thereof.

14. Compounds of the general formula

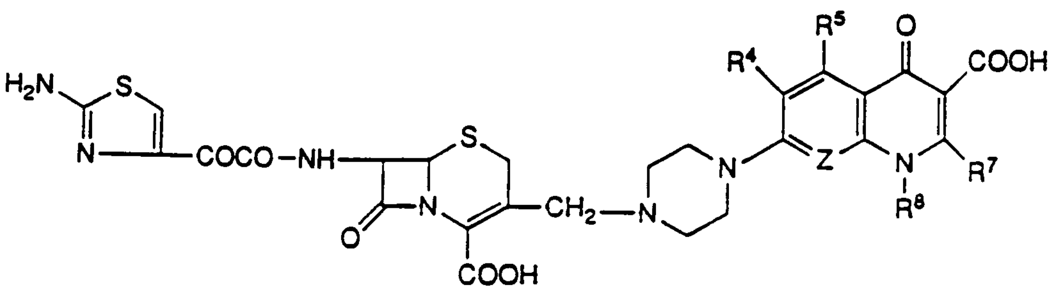

III

wherein $R^4$, $R^5$, $R^7$, $R^8$ und Z are as above, and salts thereof.

15. Compounds as set forth in any one of claims 1-13 as pharmaceutically active substances.

16. Compounds as set forth in any one of claims 1-13 as pharmaceutically active substances for the treatment and prophylaxis of infectious diseases.

17. A pharmaceutical preparation containing an end product according to any one of claims 1-13.

18. A pharmaceutical preparation for the treatment and prophylaxis of infectious diseases containing an end product according to any one of claims 1-13.

**19.** A process for the manufacture of the compounds of claim 1 which comprises
(a) reacting a compound of the general formula

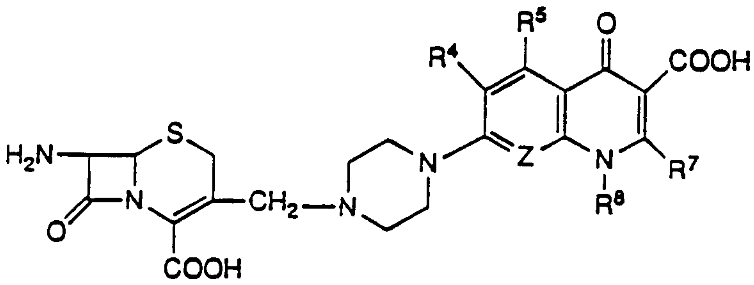

II

wherein $R^4$, $R^5$, $R^7$, $R^8$ and Z are as above, or a salt thereof with a carboxylic acid of the general formula $R^3OH$ or a reactive derivative thereof, or
(b) converting a compound corresponding to formula I, in which at least one of carboxy, amino and hydroxy groups is protected, to a compound of formula I, or
(c) for the manufacture of a compound of formula I, in which $R^3$ is group (a), reacting a compound of the general formula

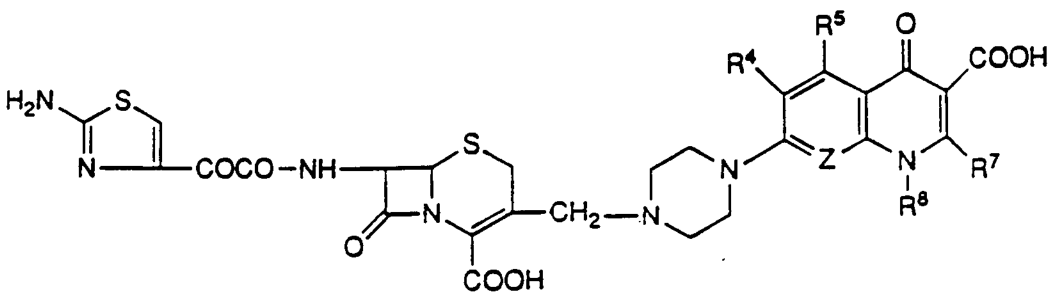

III

wherein $R^4$, $R^5$, $R^7$, $R^8$ and Z are as above, or a salt thereof with a compound of the general formula

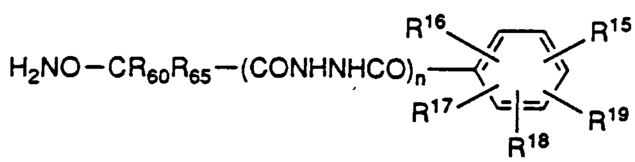

IV

wherein $R^{15}$-$R^{19}$, $R^{60}$, $R^{65}$ and n are as above, or with a salt thereof, or
(d) for the manufacture of pharmaceutically acceptable acid or base addition salts or hydrates of compounds of formula I or hydrates of such salts converting a compound of formula I into such salt or hydrate or into a hydrate of such salt.

**20.** The use of the compounds according to any one of claims 1-13 in the treatment or prophylaxis of illnesses.

**21.** The use of the compounds according to any one of claims 1-13 in the treatment or prophylaxis of infectious diseases.

**22.** The use of the compounds according to any one of claims 1-13 for the manufacture of medicaments for the treatment or prophylaxis of infectious diseases.

European Patent Office

# EUROPEAN SEARCH REPORT

Application Number

EP 92 10 0119

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 335 297 (F. HOFFMANN-LA ROCHE)<br>* Pages 1-2; pages 56-74, claims *<br>--- | 1-22 | C 07 D 501/46<br>C 07 D 501/42<br>A 61 K 31/545<br>C 07 D 501/44 |
| Y | WO-A-9 116 327 (NORWICH EATON PHARMACEUTICALS)<br>* Page 0; pages 74-81, claims *<br>--- | 1-22 | |
| Y | EP-A-0 416 410 (SHIONOGI SEIYAKU K.K.)<br>* Page 1; pages 46-47, claims *<br>--- | 1-22 | |
| Y | JOURNAL OF MEDICINAL CHEMISTRY, vol. 34, no. 2, February 1991, pages 669-675, American Chemical Society, Washington, DC, US; H.A. ALBRECHT et al.: "Dual-action cephalosporins: cephalosporin 3'-quaternary ammonium quinolones"<br>--- | 1-22 | |
| Y | EP-A-0 189 287 (NIPPON PHARMACEUTICAL DEVELOPMENT INSTITUTE CO., LTD)<br>* Pages 119-127, claims *<br>--- | 1-22 | TECHNICAL FIELDS SEARCHED (Int. Cl.5) |
| E | EP-A-0 492 277 (HOFFMANN-LA ROCHE)<br>* Complete specification *<br>----- | 1-22 | C 07 D 501/00 |

The present search report has been drawn up for all claims

| Place of search | Date of completion of the search | Examiner |
|---|---|---|
| THE HAGUE | 26-08-1992 | LUYTEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document